# EUROPEAN PATENT SPECIFICATION

(11) **EP 2 817 013 B1**
(45) Date of publication and mention of the grant of the patent: **15.05.2019**
(21) Application number: 13707782.2
(22) Date of filing: 21.02.2013
(51) Int. Cl.: A61K 31/722, A61K 47/32, A61L 26/00, A61P 17/02

(54) **ANTIMICROBIAL COMPOSITIONS, THE PREPARATION AND USE THEREOF**
ANTIMIKROBILLE ZUSAMMENSETZUNGEN, IHRE HERSTELLUNG UND VERWENDUNG
COMPOSITIONS ANTIMICROBIENNES, LEUR FABRRICATION ET LEUR UTILISATION

(30) Priority: 22.02.2012 US 201261601804 P
(43) Date of publication of application: 31.12.2014
(73) Proprietor: KCI Licensing, Inc., San Antonio, TX 78265 (US)
(72) Inventor: VAIL, Neal, San Antonio, TX 78213 (US)
(74) Representative: Ashton, Timothy
(86) International application number: PCT/US2013/027107
(87) International publication number: WO 2013/126550

(56) References cited:
- WO-A1-2011/028967
- WO-A2-2007/024972
- US-A1- 2010 056 474
- US-A1- 2010 291 169
- US-A1- 2011 054 392
- DENG, YU ET AL: "Preparation and characterization of hyaluronan/chitosan carbodiimide", POLYMER INTERNATIONAL , 56(6), 738-745 CODEN: PLYIEI; ISSN: 0959-8103, 2007, XP002697997,

## Description

The present invention is as set out in the claims. In particular, the invention is as set out in the following clauses:
1. A composition comprising a polycation and a polyanion, wherein the composition has antimicrobial activity and wherein the polycation comprises a saccharide of formula (I): 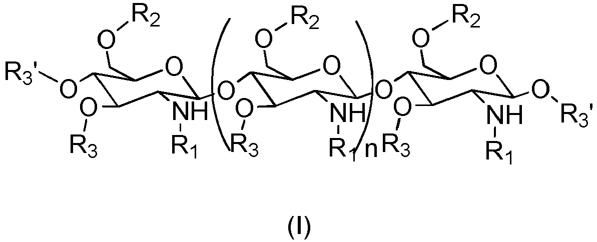 wherein:
   n is an integer ranging from 20 and 6000,
   R₁ at each occurrence independently is selected from hydrogen, alkyl, and -C(O)-R₄, wherein at least one R₁
      is selected from 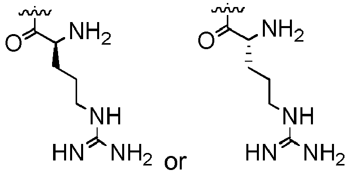
   R₂ at each occurrence independently is selected from hydrogen, alkyl, and -C(O)-R₄;
   R₃ at each occurrence independently is selected from hydrogen, alkyl, and -C(O)-R₄; and
   R₃' at each occurrence independently is selected from hydrogen, alkyl, and -C(O)-R₄;
   wherein R₄ at each occurrence independently is selected from hydrogen, alkyl, alkenyl, alkynyl alkoxy, cycloalkyl, aryl, heterocyclyl, and heteroaryl;
   or a salt thereof,
   wherein the polyanion comprises a polyacrylate having one or more pendant phosphate groups;
   wherein the polycation, the polyanion, or each of the polyanion and polycation comprises a crosslinkable group; and
   wherein the composition is biodegradable.
2. The composition of clause 1, wherein the polycation has antimicrobial activity.
3. The composition of clauses 1 or 2, wherein the polycation comprises one or more amino groups.
4. The composition of any one of clauses 1 to 3, wherein the polycation comprises a fragment of formula (III): 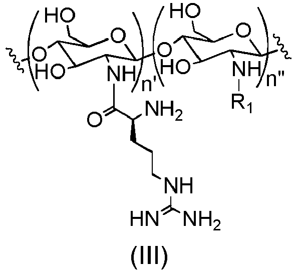 wherein:
   n' and n" each independently is an integer ranging from 0 and 6000, wherein 1 ≤ (n' + n") ≤ 6000, and
   R₁ at each occurrence independently is hydrogen or -C(O)-R₄ and R₄ is alkyl.
5. The composition of any one of clauses 1 to 4, wherein the polyanion further comprises a sulfate, a sulfonate, a borate, a boronate, or a phosphonate group.
6. The composition of any one of clauses 1 to 4, wherein the polyanion comprises a fragment of formula (VII): 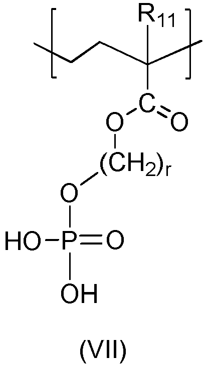 wherein:
   R₁₁ at each occurrence independently is selected from hydrogen or alkyl; and
   r at each occurrence independently is an integer from 1 to 10;
   or a pharmaceutically acceptable salt thereof.
7. The composition of clause 6, wherein at least one R₁₁ is methyl and at least one r is 2.
8. The composition of any one of clauses 1 to 7, further comprising:
   a multivalent cation; and/or,
   a reinforcing component; and/or,
   an initiator and optionally a co-initiator; and/or,
   a bioactive agent.
9. The composition of any one of clauses 1 to 8, wherein the composition is a complex coacervate and/or an adhesive complex coacervate.
10. The composition of any one of clauses 1 to 9 for use in therapy.
11. The composition of any one of clauses 1 to 9 for use in a method of treating or preventing an infection, the method comprising applying the composition of any one of clauses 1 to 9 to an infection on a subject in need thereof; optionally, wherein the infection is selected from a bacterial infection and a fungal infection.
12. The composition of any one of clauses 1 to 9 for use in a method of attaching an implant to a tissue, the method comprising applying to an implant or a tissue of a subject in need thereof the composition of any one of clauses 1 to 9, and contacting the implant with the tissue; optionally, wherein the tissue is a bone tissue, a muscle tissue, nerve tissue or an epithelial tissue.
13. The composition of any one of clauses 1 to 9 for use in a method of treating a defect, the method comprising applying the composition to a defect in a subject in need thereof; optionally, wherein the defect is selected from a bone defect, a surgical incision, a puncture, a gap between two tissue planes, and a gap between two tissues; further optionally, wherein the defect is a bone fracture, a surgical incision, a puncture in an internal organ, optionally wherein the internal organ is a blood vessel, an intestine, stomach, a kidney, bladder, uterus, lung or diaphragm, a corneal laceration or a sclera laceration.
14. A method of making the composition of any one of clauses 1 to 9 comprising the steps of (1) mixing a polycation solution and a polyanion solution, wherein the polycation has antimicrobial activity; and (2) adjusting the pH of the polycation solution or the polyanion solution to a desired value.
15. A kit for forming a composition according to any one of clauses 1 to 9, comprising a polycation and a polyanion, wherein the polycation has antimicrobial activity and wherein the polycation comprises a saccharide of formula (I): 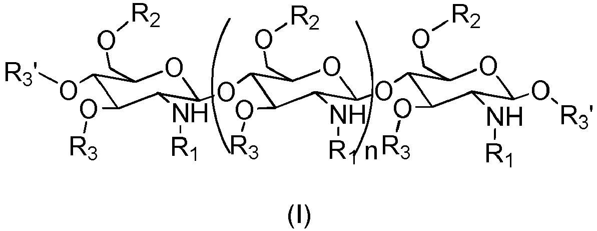 wherein:
   n is an integer ranging from 20 and 6000,
   R₁ at each occurrence independently is selected from
   hydrogen, alkyl, and -C(O)-R₄, wherein at least one R₁ is selected from 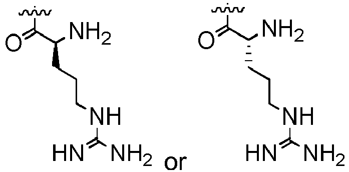
      R₂ at each occurrence independently is selected from hydrogen, alkyl, and -C(O)-R₄;
      R₃ at each occurrence independently is selected from hydrogen, alkyl, and -C(O)-R₄; and
      R₃' at each occurrence independently is selected from hydrogen, alkyl, and -C(O)-R₄;
   wherein R₄ at each occurrence independently is selected from hydrogen, alkyl, alkenyl, alkynyl alkoxy, cycloalkyl, aryl, heterocyclyl, and heteroaryl;
   or a salt thereof,
   wherein the polyanion comprises a polyacrylate having one or more pendant phosphate groups;
   wherein the polycation, the polyanion, or each of the polyanion and polycation comprises a crosslinkable group.
16. A method of making a structure comprising providing the composition of any one of clauses 1 to 9 and using a process to make a structure.
17. A structure obtainable by the method of claim 16.

The present teachings provide new compositions each comprising one or more polycations and one or more polyanions. Macromolecular compounds with ions of opposite charge form polyionic compositions, which, depending on the charge distribution and molecular weight of the compositions, precipitate from solutions, forming complex coacervates. US2010/291169 and WO2007/024972 disclose a substrate coated with a PEC, which can be used in wound dressing. The invention is defined by the claims. Thus, in one aspect, the present teachings provide complex coacervates. In addition, another aspect of the present teachings relates to preparation and use of these new compositions.

In various embodiments, the polycations and/or polyanions each also comprises crosslinkable moieties and thus can be crosslinked with one another through curing processes. Alternatively an additional cross-linking agent may be added to promote curing. These compositions can also include metal cations, reinforcing components, initiators, co-initiators, or bioactive agents, each of which is further described herein.

The compositions described herein can have several desired properties. For example, some compositions exhibit low interfacial tension in water, some have adjustable cohesive strength, some have antimicrobial activity, some are suitable for dissolution at or near physiological pH, some are capable of promoting cell attachment, some are biocompatible, and some are biodegradable. Thus, in various embodiments, the complex coacervates are used in water-based applications, for example, in applications used in the body. In various embodiments, the complex coacervates are used as adhesives. Additionally, the ability of certain complex coacervates to crosslink can increase the cohesive strength of the coacervates. In various embodiments, the compositions have antimicrobial activity.

Additionally, the present teachings also provide the use of these compositions, for example, as adhesives, sealants, hemostats, fillers, coatings, composites, flow agents, or drug delivery devices.

Reference will be made in detail to certain exemplary embodiments according to the present teachings, certain examples of which are illustrated in the accompanying drawings. Wherever possible, the same reference numbers will be used throughout the drawings to refer to the same or like parts. It will be understood that both the foregoing description and the following reference in detail to certain exemplary embodiments are not restrictive.

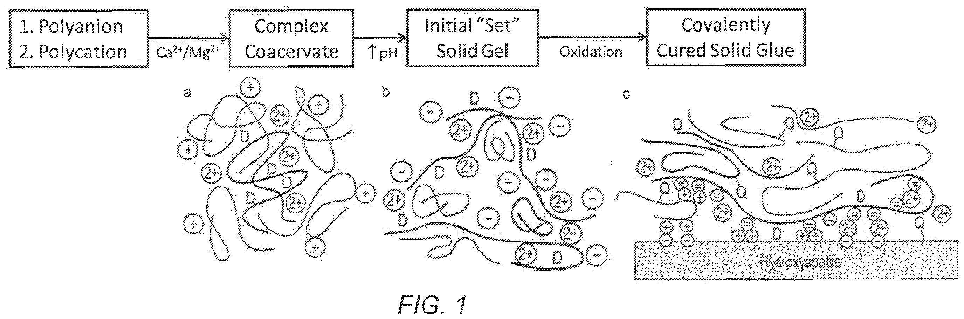

Fig. 1 illustrates an exemplary formation of a complex coacervate: (a) an arginine-modified chitosan derivative (polycation) is combined with a polyanion in the presence of metal cations; (b) a polyanion is paired with the polycation to form a first complex coacervate; (c) a second complex coacervate (*e.g.*, an "initial 'set' solid gel") is formed, for example, by changing the pH of the first complex coacervate; and (d) a third complex coacervate (*e.g*., a "covalently cured solid glue") is formed, for example, by crosslinking the second complex coacervate.

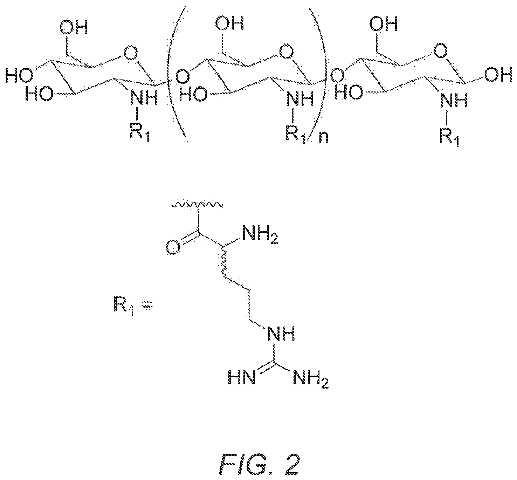

Fig 2. illustrates the structure of an exemplary arginine-modified chitosan derivative (polycation).

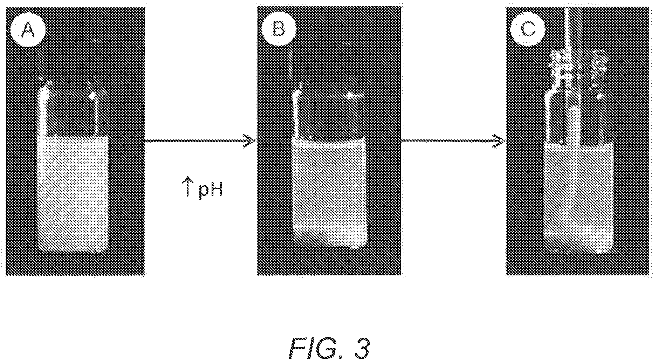

Fig 3. illustrates the formation of an exemplary complex coacervate: (a) a first complex coacervate was formed by combining polycations and polyanions; (b) a second complex coacervate was formed, for example, by raising the pH of the first complex coacervate; and (c) the second complex coacervate exhibited certain properties, for example, having a density greater than water, immiscibility in water, and the ability to adhere to an object.

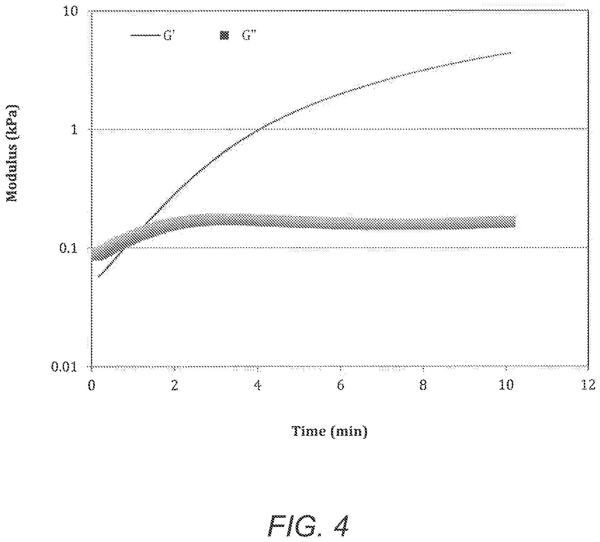

Fig 4. illustrates the complex moduli of curing chitosan derivative (CA)-containing complex coacervate using EDC as a crosslinking agent. G' is the storage modulus and G" is the loss modulus. The point where the two curves cross is the gel-point of the curing complex coacervate.

As used herein, phrases and symbols are generally intended to have the meanings as set forth below, except to the extent that the context in which they are used indicates otherwise.

The following abbreviations and terms have the indicated meanings throughout:

As used in the specification and appended claims, the articles "a," "an," and "the" include plural referents unless expressly and unequivocally limited to one referent. For example, reference to "a compound" includes a mixture of two or more compounds, and reference to "a pharmaceutically acceptable carrier" includes a mixture of one or more carriers, and the like. Accordingly, unless otherwise specified, the articles "a," "an," and "the" can have the same meanings as the term "one or more" or "at least one."

It should also be noted that the term "or" generally includes "and/or" unless the context clearly dictates otherwise.

Unless otherwise specified, the chemical groups refer to their unsubstituted and substituted forms. For example, "alkyl," unless otherwise specified, encompasses both "unsubstituted alkyl" and "substituted alkyl." By "optional" or "optionally," it is meant that the subsequently described event or circumstance may or may not occur, and that the description includes instances in which is does not. For example, "optionally substituted aryl" encompasses both "unsubstituted aryl" and "substituted aryl" as defined below. It will be understood by those skilled in the art, with respect to any group containing one or more substituents, that such groups are not intended to introduce any substitution or substitution patterns that are sterically impractical, synthetically unfeasible and/or inherently unstable.

Unless otherwise specified, the chemical groups include their corresponding monovalent, divalent, and trivalent groups. For example, methyl include monovalent methyl (-CH₃), divalent methyl (-CH₂-, methylyl), trivalent methyl 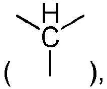 and tetravalent methyl

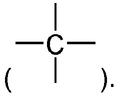

Unless otherwise specified, all numbers expressing quantities of ingredients, reaction conditions, and other properties or parameters used in the specification and claims are to be understood as being modified in all instances by the term "about." Accordingly, unless otherwise indicated, it should be understood that the numerical parameters set forth in the following specification and attached claims are approximations. At the very least, and not as an attempt to limit the application of the doctrine of equivalents to the scope of the claims, numerical parameters should be read in light of the number of reported significant digits and the application of ordinary rounding techniques.

All numerical ranges herein include all numerical values and ranges of all numerical values within the recited range of numerical values. Further, while the numerical ranges and parameters setting forth the broad scope of the disclosure are approximations as discussed above, the numerical values set forth in the Examples section are reported as precisely as possible. It should be understood, however, that such numerical values inherently contain certain errors resulting from the measurement equipment and/or measurement technique.

"Compounds" refers to compounds encompassed by structural formulae described herein and includes any specific compounds within the formulae whose structure is disclosed herein. Compounds may be identified either by their chemical structure and/or chemical name. When the chemical structure and chemical name conflict, the chemical structure is determinative of the identity of the compound. The compounds described herein may contain one or more chiral centers and/or double bonds and therefore may exist as stereoisomers such as double-bond isomers (*i.e.,* geometric isomers), enantiomers, or diastereomers. Accordingly, any chemical structures within the scope of the specification depicted, in whole or in part, with a relative configuration encompass all possible enantiomers and stereoisomers of the illustrated compounds including the stereoisomerically pure form (e.g., geometrically pure, enantiomerically pure, or diastereomerically pure) and enantiomeric and stereoisomeric mixtures. Enantiomeric and stereoisomeric mixtures can be resolved into their component enantiomers or stereoisomers using separation techniques or chiral synthesis techniques well known to the skilled artisan.

For the purposes of the present teachings, "chiral compounds" are compounds having at least one center of chirality (*i.e.,* at least one asymmetric atom, in particular at least one asymmetric C atom), having an axis of chirality, a plane of chirality, or a screw structure. "Achiral compounds" are compounds which are not chiral.

Compounds described herein include, but are not limited to, optical isomers of the polyionic and cationic compound described, racemates thereof, and other mixtures thereof. In such embodiments, the single enantiomers or diastereomers, *i.e.,* optically active forms can be obtained by asymmetric synthesis or by resolution of the racemates. Resolution of the racemates can be accomplished, for example, by conventional methods such as crystallization in the presence of a resolving agent, or chromatography, using, for example a chiral high-pressure liquid chromatography (HPLC) column. However, unless otherwise stated, it should be assumed that the polyionic and cationic compounds described herein cover all asymmetric variants, including isomers, racemates, enantiomers, diastereomers, and other mixtures thereof. In addition, the polyionic and cationic compounds described herein include Z- and E-forms (*e.g*., cis- and trans-forms) of compounds with double bonds. In embodiments in which the polyionic and cationic compounds described herein exist in various tautomeric forms, compounds provided by the present disclosure include all tautomeric forms of the compound.

The term "alkyl" as used herein refers to a saturated straight or branched hydrocarbon, such as a straight or branched group of 1 to 22, 1 to 8, or 1 to 6 carbon atoms, referred to herein as (C₁-C₂₂)alkyl, (C₁-C₈)alkyl, and (C₁-C₆)alkyl, respectively. The alkyl groups can be substituted with one or more groups each independently selected from alkyl, alkenyl, alkynyl, aryl, arylalkyl, alkoxy, aryloxy, cycloalkyl, heteroaryl, heterocyclyl, halogen, cyano, hydroxyl, oxo, amino, imino, phosphate, sulfide, sulfinyl, sulfonyl, and sulfonic acid and each of the alkyl, alkenyl, alkynyl, aryl, arylalkyl, alkoxy, aryloxy, cycloalkyl, heteroaryl, and heterocyclyl optionally can be substituted with one or more suitable substituents as described herein. Exemplary alkyl groups include, but are not limited to, methyl, ethyl, propyl, isopropyl, 2-methyl-1-propyl, 2-methyl-2-propyl, 2-methyl-1-butyl, 3-methyl-1-butyl, 2-methyl-3-butyl, 2,2-dimethyl-1-propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, butyl, isobutyl, t-butyl, pentyl, isopentyl, neopentyl, hexyl, heptyl, octyl, etc.

The term "alkenyl" as used herein refers to an unsaturated straight or branched hydrocarbon having at least one carbon-carbon double bond, such as a straight or branched group of 2 to 22, 2 to 8, or 2 to 6 carbon atoms, referred to herein as (C₂-C₂₂)alkenyl, (C₂-C₈)alkenyl, and (C₂-C₆)alkenyl, respectively. The alkenyl groups can be substituted with one or more groups each independently selected from alkyl, alkenyl, alkynyl, aryl, arylalkyl, alkoxy, aryloxy, cycloalkyl, heteroaryl, heterocyclyl, halogen, cyano, hydroxyl, oxo, amino, imino, phosphate, sulfide, sulfinyl, sulfonyl, and sulfonic acid and each of the alkyl, alkenyl, alkynyl, aryl, arylalkyl, alkoxy, aryloxy, cycloalkyl, heteroaryl, and heterocyclyl optionally can be substituted with one or more suitable substituents as described herein. Exemplary alkenyl groups include, but are not limited to, vinyl, allyl, butenyl, pentenyl, hexenyl, butadienyl, pentadienyl, hexadienyl, 2-ethylhexenyl, 2-propyl-2-butenyl, 4-(2-methyl-3-butene)-pentenyl, etc.

The term "alkynyl" as used herein refers to an unsaturated straight or branched hydrocarbon having at least one carbon-carbon triple bond, such as a straight or branched group of 2 to 22, 2 to 8, or 2 to 6 carbon atoms, referred to herein as (C₂-C₂₂)alkynyl, (C₂-C₈)alkynyl, and (C₂-C₆)alkynyl, respectively. The alkynyl groups can be substituted with one or more groups each independently selected from alkyl, alkenyl, alkynyl, aryl, arylalkyl, alkoxy, aryloxy, cycloalkyl, heteroaryl, heterocyclyl, halogen, cyano, hydroxyl, oxo, amino, imino, phosphate, sulfide, sulfinyl, sulfonyl, and sulfonic acid and each of the alkyl, alkenyl, alkynyl, aryl, arylalkyl, alkoxy, aryloxy, cycloalkyl, heteroaryl, and heterocyclyl optionally can be substituted with one or more suitable substituents as described herein. Exemplary alkynyl groups include, but are not limited to, ethynyl, propynyl, butynyl, pentynyl, hexynyl, methylpropynyl, 4-methyl-1-butynyl, 4-propyl-2-pentynyl, and 4-butyl-2-hexynyl, etc.

The term "alkoxy" as used herein refers to an alkyl group attached to an oxygen (-O-alkyl). "Alkoxy" groups also include an alkenyl group attached to an oxygen ("alkenyloxy") or an alkynyl group attached to an oxygen ("alkynyloxy") groups. Exemplary alkoxy groups include, but are not limited to, groups with an alkyl, alkenyl or alkynyl group of 1 to 22, 1 to 8, or 1 to 6 carbon atoms, referred to herein as (C₁-C₂₂)alkoxy, (C₁-C₈)alkoxy, and (C₁-C₆)alkoxy, respectively. The alkoxy groups can be substituted with one or more groups each independently selected from alkyl, alkenyl, alkynyl, aryl, arylalkyl, alkoxy, aryloxy, cycloalkyl, heteroaryl, heterocyclyl, halogen, cyano, hydroxyl, oxo, amino, imino, phosphate, sulfide, sulfinyl, sulfonyl, and sulfonic acid and each of the alkyl, alkenyl, alkynyl, aryl, arylalkyl, alkoxy, aryloxy, cycloalkyl, heteroaryl, and heterocyclyl optionally can be substituted with one or more suitable substituents as described herein. Exemplary alkoxy groups include, but are not limited to methoxy, ethoxy, etc.

The term "aryl" as used herein refers to a mono-, bi-, or other multi-carbocyclic, aromatic ring system. The aryl group can optionally be fused to one or more rings selected from aryls, cycloalkyls, and heterocyclyls. The aryl groups can be substituted with one or more groups each independently selected from alkyl, alkenyl, alkynyl, aryl, arylalkyl, alkoxy, aryloxy, cycloalkyl, heteroaryl, heterocyclyl, halogen, cyano, hydroxyl, oxo, amino, imino, phosphate, sulfide, sulfinyl, sulfonyl, and sulfonic acid and each of the alkyl, alkenyl, alkynyl, aryl, arylalkyl, alkoxy, aryloxy, cycloalkyl, heteroaryl, and heterocyclyl optionally can be substituted with one or more suitable substituents as described herein. Exemplary aryl groups include, but are not limited to, phenyl, tolyl, anthracenyl, fluorenyl, indenyl, azulenyl, and naphthyl, as well as benzo-fused carbocyclic moieties such as 5,6,7,8-tetrahydronaphthyl. Exemplary aryl groups also include, but are not limited to, a monocyclic aromatic ring system, wherein the ring comprises 6 carbon atoms, referred to herein as "(C₆)aryl."

The term "arylalkyl" as used herein refers to an alkyl group having at least one aryl substituent, *e.g.,* aryl-alkyl-. Exemplary arylalkyl groups include, but are not limited to, arylalkyls having a monocyclic aromatic ring system, wherein the ring comprises 6 carbon atoms, referred to herein as "(C₆)arylalkyl." The term "benzyl" as used herein refers to the group phenyl-CH₂-.

The term "aryloxy" as used herein refers to an aryl group attached to an oxygen atom. Exemplary aryloxy groups include, but are not limited to, aryloxys having a monocyclic aromatic ring system, wherein the ring comprises 6 carbon atoms, referred to herein as "(C₆)aryloxy" or phenoxy.

The term "cycloalkyl" as used herein refers to a saturated or unsaturated cyclic, bicyclic, or bridged bicyclic hydrocarbon group of 3-12 carbons or 3-8 carbons, referred to herein as "(C₃-C₁₂)cycloalkyl" and "(C₃-C₃)cycloalkyl," respectively, derived from a cycloalkane. The cycloalkyl groups can be substituted with one or more groups each independently selected from alkyl, alkenyl, alkynyl, aryl, arylalkyl, alkoxy, aryloxy, cycloalkyl, heteroaryl, heterocyclyl, halogen, cyano, hydroxyl, oxo, amino, imino, phosphate, sulfide, sulfinyl, sulfonyl, and sulfonic acid and each of the alkyl, alkenyl, alkynyl, aryl, arylalkyl, alkoxy, aryloxy, cycloalkyl, heteroaryl, and heterocyclyl optionally can be substituted with one or more suitable substituents as described herein. Exemplary cycloalkyl groups include, but are not limited to, cyclohexanes, cyclohexenes, cyclopentanes, and cyclopentenes. Cycloalkyl groups can be fused to other cycloalkyl saturated or unsaturated, aryl, or heterocyclyl groups.

The term "heteroaryl" as used herein refers to a mono-, bi-, or multi-cyclic, aromatic ring system containing one or more heteroatoms, for example 1 to 3 heteroatoms, such as nitrogen, oxygen, and sulfur. The heteroaryl groups can be substituted with one or more groups each independently selected from alkyl, alkenyl, alkynyl, aryl, arylalkyl, alkoxy, aryloxy, cycloalkyl, heteroaryl, heterocyclyl, halogen, cyano, hydroxyl, oxo, amino, imino, phosphate, sulfide, sulfinyl, sulfonyl, and sulfonic acid and each of the alkyl, alkenyl, alkynyl, aryl, arylalkyl, alkoxy, aryloxy, cycloalkyl, heteroaryl, and heterocyclyl optionally can be substituted with one or more suitable substituents as described herein. Heteroaryls can also be fused to non-aromatic rings. Illustrative examples of heteroaryl groups include, but are not limited to, pyridinyl, pyridazinyl, pyrimidyl, pyrazyl, triazinyl, pyrrolyl, pyrazolyl, imidazolyl, (1,2,3)- and (1,2,4)-triazolyl, pyrazinyl, pyrimidilyl, tetrazolyl, furyl, thienyl, isoxazolyl, thiazolyl, furyl, isoxazolyl, and oxazolyl. Exemplary heteroaryl groups include, but are not limited to, a monocyclic aromatic ring, wherein the ring comprises 2 to 5 carbon atoms and 1 to 3 heteroatoms, referred to herein as "(C₂-C₅)heteroaryl."

The terms "heterocycle," "heterocyclyl," or "heterocyclic" as used herein refer to a saturated or unsaturated 3-, 4-, 5-, 6- or 7-membered ring containing one, two, or three heteroatoms independently selected from nitrogen, oxygen, and sulfur. Heterocycles can be aromatic (heteroaryls) or non-aromatic. The heterocycle, heterocyclyl, or heterocyclic groups can be substituted with one or more groups each independently selected from alkyl, alkenyl, alkynyl, aryl, arylalkyl, alkoxy, aryloxy, cycloalkyl, heteroaryl, heterocyclyl, halogen, cyano, hydroxyl, oxo, amino, imino, phosphate, sulfide, sulfinyl, sulfonyl, and sulfonic acid and each of the alkyl, alkenyl, alkynyl, aryl, arylalkyl, alkoxy, aryloxy, cycloalkyl, heteroaryl, and heterocyclyl optionally can be substituted with one or more suitable substituents as described herein. Heterocycles also include bicyclic, tricyclic, and tetracyclic groups in which any of the above heterocyclic rings is fused to one or two rings independently selected from aryls, cycloalkyls, and heterocycles.

The term "amine" or "amino" as used herein refers to the form -NR_{d}Rₑ, where R_{d} and Rₑ independently are selected from hydrogen, alkyl, alkenyl, alkynyl, aryl, arylalkyl, cycloalkyl, and heterocyclyl. The amino can be attached to the parent molecular group through the nitrogen. The amino also may be cyclic, for example any two of R_{d} and Rₑ may be joined together or with the N to form a 3- to 12-membered ring, *e.g.,* morpholino or piperidinyl. The term amino also includes the corresponding quaternary ammonium salt of any amino group, for example, -(NR_{d}RₑR_{f})⁺, where R_{d}, Rₑ, and R_{f} independently are selected from hydrogen, alkyl, alkenyl, aryl, arylalkyl, cycloalkyl, and heterocyclyl. Exemplary amino groups include alkylamino groups, wherein at least one of R_{d} and Rₑ is an alkyl group. Each of the alkyl, alkenyl, aryl, arylalkyl, cycloalkyl, and heterocyclyl can be substituted with at least one suitable substituent as further described below.

The terms "halo" or "halogen" or "hal" as used herein refers to F, CI, Br, and I. The term "halide" as used herein refers to F, CI, Br, I, or an ionic form thereof. For example, chloride can mean -Cl or Cl⁻.

The term "cyano" as used herein refers to -CN. The term "nitro" as used herein refers to -NO₂.

The term "oxo" as used herein refers to =O.

The terms "hydroxyl" as used herein refers to -OH.

The term "imine" or "imino" as used herein refers to =NR_{g}, where R_{g} is selected from hydrogen, alkyl, alkenyl, aryl, arylalkyl, cycloalkyl, and heterocyclyl. Each of the alkyl, alkenyl, aryl, arylalkyl, cycloalkyl, and heterocyclyl can be substituted with at least one suitable substituent as further described below.

The term "phosphate" as used herein refers to the structure -OP(O)(OH)₂ or any one of its corresponding salts (*e.g*., -OP(O)(OH)ONa, -OP(O)(O)₂Na₂, -OP(O)(OH)OK, -OP(O)(O)₂K₂, etc.).

The term "phosphonate" as used herein refers to the structure -P(O)(OH)₂, -P(O)(ORₙ)OH, or any one of their corresponding salts (*e.g*., -P(O)(OH)ONa, -P(O)(O)₂Na₂, -P(O)(OH)OK, -P(O)(O)₂K₂, -P(O)(ORₕ)ONa, -P(O)(ORₕ)OK, -P(O)(O)₂K₂, etc.), where Rₕ is alkyl, alkenyl, alkynyl, aryl, arylalkyl, cycloalkyl, heteroaryl, or heterocyclyl. In some embodiments, Rₕ is alkyl, alkenyl, aryl, arylalkyl, or cycloalkyl.

The term "sulfate" as used herein refers to -OSO₂OH or any one of its corresponding salts (*e.g.,* -OSO₃Na, -OSO₃K, etc.).

The term "sulfonate" as used herein refers to -SO₂OH (also known as "sulfonic acid") or any one of its corresponding salts (*e.g*., -SO₃Na, -SO₃K, etc.).

The term "borate" as used herein refers to -OB(OH)₂ or salts thereof.

The term "boronate" as used herein refers to -B(OH)₂, -B(ORᵢ)(OH) or salts thereof where R₁ is alkyl, alkenyl, alkynyl, aryl, arylalkyl, cycloalkyl, heteroaryl, or heterocyclyl.

Each of "suitable substituents" referred to herein is selected from alkyl, alkenyl, alkynyl, aryl, heteroaryl, cycloalkyl, alkoxy, aryloxy, cyano, hydroxyl, oxo, imino, halo, and amino. In some embodiments, each of the alkyl, alkenyl, and alkynyl described herein comprises 1 to 22, 1 to 8, or 1 to 6 carbon atoms. In some embodiments, the cycloalkyl described herein comprises 3 to 7 ring carbon atoms. In some embodiments, the alkoxy described herein comprises 1 to 22, 1 to 8, or 1 to 6 carbon atoms. In some embodiments, the aryloxy is phenoxy. In some embodiments, the amino is selected from -NH(C₁₋₂₂, C₁₋₈, or C₁₋₆ alkyl), -N(C₁₋₂₂, C₁₋₈, and C₁₋₆ alkyl)₂, -NH(pheny), and -N(phenyl)₂. One of skill in art can readily choose a suitable substituent based on the stability and synthetic activity of the compounds of the present teachings.

Accordingly, alkyl can be substituted with one or more hydroxyl groups to form "hydroxylalkyl," including 2-hydroxylpropyl 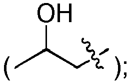 aryl group can be substituted with one or more hydroxyl groups to form "hydroxylaryl"; alkenyloxy (an alkoxy) can be substituted with an oxo to form unsubstituted and substituted acryloxy groups (*e.g.*, acryloxy 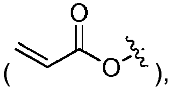 methacryloxy 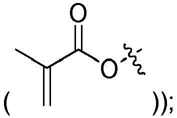 and methylamino (an amino) can be substituted with an amino group and an imino group to form guanidino

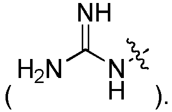

The term "pharmaceutically acceptable salt(s)" refers to salts of acidic or basic groups that may be present in compounds used in the present compositions. Compounds included in the present teachings that are basic in nature are capable of forming a wide variety of salts with various inorganic and organic acids. The acids that may be used to prepare pharmaceutically acceptable acid addition salts of such basic compounds are those that form non-toxic acid addition salts, *i.e.,* salts containing pharmacologically acceptable anions, including but not limited to sulfate, citrate, matate, acetate, oxalate, chloride, bromide, iodide, nitrate, sulfate, bisulfate, phosphate, acid phosphate, isonicotinate, acetate, lactate, salicylate, citrate, tartrate, oleate, tannate, pantothenate, bitartrate, ascorbate, succinate, maleate, gentisinate, fumarate, gluconate, glucaronate, saccharate, formate, benzoate, glutamate, methanesulfonate, ethanesulfonate, benzenesulfonate, *p*-toluenesulfonate and pamoate (*i.e.,* 1,1'-methylene-bis-(2-hydroxy-3-naphthoate)) salts. Compounds included in the present teachings that include an amino moiety may form pharmaceutically acceptable salts with various amino acids, in addition to the acids mentioned above. Compounds included in the present compositions that are acidic in nature are capable of forming base salts with various pharmacologically acceptable cations. Examples of such salts include alkali metal or alkaline earth metal salts and, particularly, calcium, magnesium, sodium, lithium, zinc, potassium, and iron salts.

"Antimicrobial activity" used herein means the ability to kill or inhibit the growth of microorganisms, including bacteria, yeasts, fungi, and protozoan.

The present teachings provide new compositions and applications thereof. The compositions of the present teachings comprise a polycation and a polyanion and have antimicrobial activity.

In various embodiments, the new compositions are complex coacervates, described in some instances as associative liquids in which the individual polymer components can diffuse throughout the entire phase. As described above, in some instances, some complex coacervates exhibit low interfacial tension with water and hydrophilic substrates. For example, when applied to substrates in an aqueous environment, a complex coacervate of the present teachings can spread evenly over the interface and penetrate cracks and defects. In some embodiments, upon intermolecular crosslinking, the complex coacervate forms a strong, insoluble, cohesive material. In some embodiments, the new compositions have analgesic effects, antitumor activity, anticoagulant activity, anticholesterolemic activity, antimicrobial activity, or antioxidative activity. The new compositions have antimicrobial activity.

Exemplary components used to prepare a complex coacervate of the present teachings and exemplary methods for making and using the same are described below.

The polycation described herein generally comprises a polymer backbone with a plurality of cationic groups at a particular pH. The cationic groups can be pendant to the polymer backbone and/or incorporated within the polymer backbone. In various embodiments, the polycation is any biocompatible polymer possessing cationic groups or groups that can be converted to cationic groups, for example, by adjusting the pH. In various embodiments, the polycation has analgesic effects, antitumor activity, anticoagulant activity, anticholesterolemic activity, antimicrobial activity, and/or antioxidative activity. In some embodiments, the polycation has antimicrobial activity.

The polycation is a polyamine compound. The amino groups of the polyamine can be branched or part of the polymer backbone. The amino group can be a primary, secondary, or tertiary amino group that can be protonated to produce a cationic ammonium group at a selected pH. As discussed above, the amino group can be further substituted with one or more
suitable substituents. In particular embodiments, the amino group can be substituted with an imino group (*e.g.*, unsubstituted or substituted guanidine). The amino group can also include quaternary ammonium group.

In general, the polyamine is a polymer with a large excess of positive charges relative to negative charges at the relevant pH, as reflected in its isoelectric point (pi), which is the pH at which the polymer has a net neutral charge. The number of amino groups present on the polycation ultimately determines the charge of the polycation at a particular pH. For example, the polycation can have from 10 to 90 mole %, 10 to 80 mole %, 10 to 70 mole %, 10 to 60 mole %, 10 to 50 mole %, 10 to 40 mole %, 10 to 30 mole %, or 10 to 20 mole % amino groups. In some embodiments, the polyamine has an excess positive charge at a pH of about 7, with a pl significantly greater than 7.

The amino group can be derived from a residue of lysine, histidine, or arginine attached to the polycation. Any anionic counterions can be used in association with the cationic polymers. The counterions should be physically and chemically compatible with the essential components of the composition and do not otherwise unduly impair product performance, stability or aesthetics. Non-limiting examples of such counterions include halides (*e.g.,* chloride, fluoride, bromide, iodide), sulfate and methylsulfate.

The polycation is a biodegradable polyamine. The biodegradable polyamine can be a synthetic polymer or naturally-occurring polymer. The mechanism by which the polyamine can degrade will vary depending upon the polyamine that is used. In the case of natural polymers, without wishing to be bound by any particular theory, they can be biodegradable because there are enzymes that can hydrolyze the polymers and break the polymer chain. For example, proteases can hydrolyze natural proteins like gelatin. In the case of synthetic biodegradable polyamines, they can also possess chemically labile bonds. For example, β-aminoesters have hydrolyzable ester groups. In addition to the nature of the polyamine, other considerations such as the molecular weight of the polyamine and crosslink density of the adhesive can be varied in order to modify the degree of biodegradability.

The biodegradable polyamine is a saccharide. Thus, saccharides bearing one or more amino groups can be used herein. The saccharides described herein can be monosaccharides, disaccharides, oligosaccharides, or polysaccharides. For example, the saccharides described herein have antimicrobial activity. The saccharides can be oligosaccharides or polysaccharides. The saccharide may be chitosan or chemically modified chitosan.

The polycation is a saccharide of Formula I, or a salt thereof: 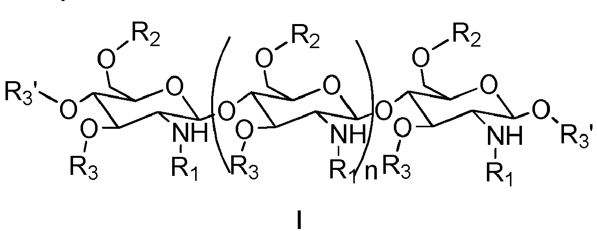 wherein:
n is an integer ranging from 20 and 6000,
R₁ at each occurrence independently is selected from hydrogen, alkyl, and -C(O)-R₄;
R₂ at each occurrence independently is selected from hydrogen, alkyl, and -C(O)-R₄;
R₃ at each occurrence independently is selected from hydrogen, alkyl, and -C(O)-R₄; and
R₃' at each occurrence independently is selected from hydrogen, alkyl, and -C(O)-R₄;
wherein R₄ at each occurrence independently is selected from hydrogen, alkyl, alkenyl, akynyl, alkoxy, cycloalkyl, aryl, heterocyclyl, and heteroaryl.

In some embodiments, the salt is a pharmaceutically acceptable salt.

In some embodiments, at least one R₁ is hydrogen. In some embodiments, at least one R₁ is alkyl. For example, R₁ can be methyl, ethyl, propyl, or isopropyl. In some embodiments, at least one R₁ is -C(O)-R₄, where R₄ is defined as herein.

In some embodiments, at least one R₂ is hydrogen. In some embodiments, at least one R₂ is alkyl. For example, R₂ can be methyl, ethyl, propyl, or isopropyl. In some embodiments, at least one R₂ is -C(O)-R₄, where R₄ is defined as herein.

In some embodiments, at least one R₃ is hydrogen. In some embodiments, at least one R₃ is alkyl. For example, R₃ can be methyl, ethyl, propyl, or isopropyl. In some embodiments, at least one R₃ is -C(O)-R₄, where R₄ is defined as herein.

In some embodiments, at least one R₃' is hydrogen. For example, each R₃' is hydrogen.

For example, R₄ can be alkyl, alkenyl, or alkoxy, each of which optionally is substituted with one or more suitable substituents. In certain embodiments, R₄ is alkyl. In particular embodiments, R₄ is methyl. Accordingly, R₁ can be acetyl (CH₃C(O)-). In particular embodiments, R₄ is alkyl substituted with one or more groups independently selected from alkyl, alkenyl, akynyl, alkoxy, oxo, imino, amino, cycloalkyl, aryl, heterocyclyl, and heteroaryl, each of which optionally is substituted with one or more suitable substituents.

In various aspects of the present disclosure, at least one R₁ has Formula II: 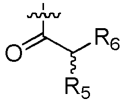 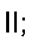 wherein:
R₅ independently is hydrogen, alkyl, alkenyl, alkoxy, amino, cycloalkyl, aryl, or heterocyclyl; and
R₆ independently is hydrogen, alkyl, alkenyl, or amino.

In various aspects of the present disclosure, R₅ independently is alkyl, alkenyl, alkoxy, amino, or cycloalkyl, each of which optionally is substituted with one or more suitable substituents. For example, R₅ independently can be alkyl optionally substituted with one or more groups independently selected from alkoxy, amino, and aryl, each of which optionally is substituted with one or more suitable substituents. In some aspects of the present disclosure, R₅ is alkyl substituted with amino, which is further substituted with one or more groups each independently selected from amino, alkoxy, oxo, and imino. In certain aspects of the present disclosure, R₅ is alkyl substituted with guanidino.

In various aspects of the present disclosure, R₆ independently is hydrogen or amino. In some aspects of the present disclosure, R₆ is amino. In certain aspects of the present disclosure, R₆ is unsubstituted amino (-NH₂).

At least one R₁ is selected from 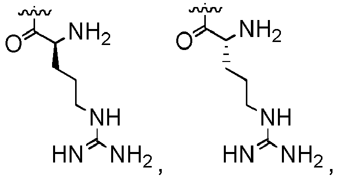 and combinations thereof.

Thus, in certain embodiments, the polycation comprises a fragment of Formula III: 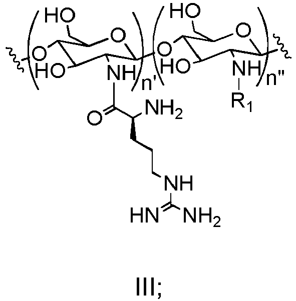 wherein:
n' and n" each independently is an integer ranging from 0 and 6000,
1 ≤ (n' + n") ≤ 6000, and
R₁ at each occurrence independently is hydrogen or -C(O)-R₄, where R₄ is defined herein.

For example, R₄ can be alkyl, including methyl. Thus, in certain embodiments, R₄ is methyl and, accordingly, R₁ is acetyl. In certain embodiments, at least one R₁ is hydrogen.

In various embodiments, n is in the range of 20 and 6000. In some embodiments, n is in the range of 20 and 3000, 20 and 1000, 20 and 500, 20 and 200, 20 and 150, and 20 and 100. In certain embodiments, n is in the range of 20 and 200. In certain embodiments, n is in the range of 20 and 150. In certain embodiments, n is in the range of 20 and 100.

In various embodiments, the sum of n' and n" is in the range of 1 and 6000. In some embodiments, the sum of n' and n" is in the range of 1 and 3000, 1 and 1000, 1 and 500, 1 and 200, 1 and 150, and 1 and 100. In certain embodiments, the sum of n' and n" is in the range of 1 and 200. In certain embodiments, the sum of n' and n" is in the range of 1 and 150. In certain embodiments, the sum of n' and n" is in the range of 1 and 100.

Further, the ratio of n' to n" can range from about 1:100 to about 100:1. For example, the ratio of n' to n" can be about 1:10, about 1:5, about 1:4, about 1:2, about 1:1, about 2:1, about 4:1, about 5:1, or about 10:1. In some aspects of the present disclosure, the ratio of n' to n" is about 1:5. In some aspects of the present disclosure, the ratio of n' to n" is about 1:4. In some aspects of the present disclosure, the ratio of n' to n" is about 1:2. In some aspects of the present disclosure, the ratio of n' to n" is about 1:1. In some aspects of the present disclosure, the ratio of n' to n" is about 2:1. In some aspects of the present disclosure, the ratio of n' to n" is about 4:1. In some aspects of the present disclosure, the ratio of n' to n" is about 5:1.

In various aspects of the present disclosure, the polycation is a peptide. For example, the polycation is an antimicrobial peptide. The peptide can be a dipeptide, a tripeptide, a tetrapeptide, an oligopeptide, and a polypeptide. In some aspects of the present disclosure, the peptide is an oligopeptide or a polypeptide. In certain aspects of the present disclosure, the peptide is selected from cathelicidins, cecropins, defensins, dermcidins, histatins, magainins, melittins, protegrins, polymyxins, tachyplesins, and thionins.

In various aspects of the present disclosure, the polycation comprises one or more quaternary ammonium groups. For example, the polycations having one or more quaternary ammonium groups have antimicrobial activity. The quaternary ammonium groups independently can be part of the polymer back bone or a pendant of the polymer back bone. In some aspects of the present disclosure, the polycation has Formula IV or a salt thereof: 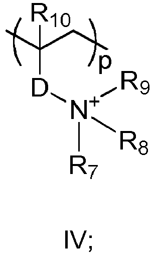 wherein
D is a divalent group selected from alkyl, aryl, arylalkyl, and alkoxy;
R₇, R₈, and R₉ each at each occurrence independently is hydrogen, alkyl, alkoxy, amino, alkenyl, alkynyl, aryl, arylalkyl, cycloalkyl, or heterocyclyl;
or one of R₇, R₈, and R₉ is connected with the polymeric back bone to form a heterocyclyl;
or two of R₇, R₈, and R₉ are connected to each other to form a heterocyclyl;
   R₁₀ at each occurrence is hydrogen or alkyl; and
   p is an integer in the range of 20 and 6000.

In some aspects of the present disclosure, the salt is a pharmaceutically acceptable salt.

In various aspects of the present disclosure, D is a divalent group selected from alkyl, arylalkyl, and alkoxy. In some aspects of the present disclosure, D is divalent alkyl optionally substituted with one or more groups each selected from hydroxyl, alkoxy, amino, oxo, and halide, where alkoxy optionally is substituted with one or more groups selected from hydroxyl, alkoxy, amino, and oxo. In certain aspects of the present disclosure, D is divalent methyl (or sometimes known as methylyl). In certain aspects of the present disclosure, D is divalent alkyl substituted with oxo and divalent alkoxy, where the alkoxy optionally is substituted with hydroxyl. In particular aspects of the present disclosure, D is -CO(O)-(CH₂)₂- or -CO(O)-CH₂-CH(OH)-CH₂-. In some aspects of the present disclosure, D is divalent arylakyl optionally substituted with one or more groups each selected from alkyl, hydroxyl, alkoxy, amino, and halide. In certain aspects of the present disclosure, D is unsubstituted divalent arylalkyl. In particular aspects of the present disclosure, D is divalent benzyl.

In various aspects of the present disclosure, R₇, R₈, and R₉ each at each occurrence independently is hydrogen, alkyl, alkoxy, amino, arylalkyl, cycloalkyl, or heterocyclyl. For example, at least one of R₇, R₈, and R₉ is hydrogen. In some aspects of the present disclosure, at least one R₇, R₈, and R₉ at each occurrence independently is alkyl. For example, at least one R₇, R₈, and R₉ is methyl. In certain aspects of the present disclosure, R₇, R₈, and R₉ each is methyl.

In some aspects of the present disclosure, one of R₇, R₈, and R₉ is divalent alkyl that is connected with the polymer back bone. In certain aspects of the present disclosure, one of R₇, R₈, and R₉ is divalent methyl (methylyl) that is connected with the polymer back bone. Each of the remaining two of R₇, R₈, and R₉, for example, is methyl. Accordingly, the polycation can have Formula V: 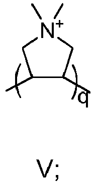 wherein q an integer in the range of 20 and 6000.

In various aspects of the present disclosure, at least one R₁₀ is hydrogen. In various aspects of the present disclosure, at least one R₁₀ is methyl. In some aspects of the present disclosure, the polycation is selected from poly(diallyldimethylammonium halide), poly(vinylbenzyltrimethylammonium halide), poly(acryloxyethyltrimethylammonium halide), poly(methacryloxyethyltrimethylammonium halide), poly(methacryloxy-2-hydroxypropyltrimethylammonium halide), and a co-polymer thereof. In certain embodiments, the polycation is poly(diallyldimethylammonium chloride) (PDADMAC).

In other aspects of disclosure, the polycation is a micelle or mixed micelle formed with cationic surfactants. The cationic surfactant can be mixed with nonionic surfactants to create micelles with variable charge ratios. The micelles are polycationic by virtue of the hydrophobic interactions that form a polyvalent micelle.

Examples of nonionic surfactants include the condensation products of a higher aliphatic alcohol, such as a fatty alcohol, containing about 8 to about 20 carbon atoms, in a straight or branched chain configuration, condensed with about 3 to about 100 moles, preferably about 5 to about 40 moles, most preferably about 5 to 20 moles of ethylene oxide. Examples of such nonionic ethoxylated fatty alcohol surfactants are the Tergitol™ 15-S series from Union Carbide and Brij™ surfactants from ICI. Tergilol™ 15-S surfactants include C₁₁-C₁₅ secondary alcohol polyethyleneglycol ethers. Brij™97 surfactant is polyoxyethylene(10) oleyl ether; Brij™58 surfactant is polyoxyethylene(20) cetyl ether; and Brij™ 76 surfactant is polyoxyethylene(10) stearyl ether.

Another useful class of nonionic surfactants includes the polyethylene oxide condensates of one mole of alkyl phenol containing from about 6 to 12 carbon atoms in a straight or branched chain configuration, with ethylene oxide. Examples of nonreactive nonionic surfactants are the Igepal™ CO and CA series from Rhone-Poulenc. Igepal™CO surfactants include nonylphenoxy poly(ethyleneoxy)ethanols. Igepal™ CA surfactants include octylphenoxy poly(ethyleneoxy)ethanols.

Another useful class of hydrocarbon nonionic surfactants includes block copolymers of ethylene oxide and propylene oxide or butylene oxide. Examples of such nonionic block copolymer surfactants are the Pluronic™ and Tetronic™ series of surfactants from BASF. Pluronic™ surfactants include ethylene oxide-propylene oxide block copolymers. Tetronic™ surfactants include ethylene oxide-propylene oxide block copolymers.

In other embodiments, the nonionic surfactants include sorbitan fatty acid esters, polyoxyethylene sorbitan fatty acid esters and polyoxyethylene stearates. Examples of such fatty acid ester nonionic surfactants are the Span™, Tween™, and Myj™ surfactants from ICI. Span™ surfactants include C₁₂-C₁₈ sorbitan monoesters. Tween™ surfactants include poly(ethylene oxide) C₁₂-C₁₈ sorbitan monoesters. Myj™ surfactants include poly(ethylene oxide) stearates.

In some aspects of disclosure, the nonionic surfactant can include polyoxyethylene alkyl ethers, polyoxyethylene alkyl-phenyl ethers, polyoxyethylene acyl esters, sorbitan fatty acid esters, polyoxyethylene alkylamines, polyoxyethylene alkylamides, polyoxyethylene lauryl ether, polyoxyethylene cetyl ether, polyoxyethylene stearyl ether, polyoxyethylene oleyl ether, polyoxyethylene octylphenyl ether, polyoxyethylene nonylphenyl ether, polyethylene glycol laurate, polyethylene glycol stearate, polyethylene glycol distearate, polyethylene glycol oleate, oxyethylene-oxypropylene block copolymer, sorbitan laurate, sorbitan stearate, sorbitan distearate, sorbitan oleate, sorbitan sesquioleate, sorbitan trioleate, polyoxyethylene sorbitan laurate, polyoxyethylene sorbitan stearate, polyoxyethylene sorbitan oleate, polyoxyethylene laurylamine, polyoxyethylene laurylamide, laurylamine acetate, hard beef tallow propylenediamine dioleate, ethoxylated tetramethyldecynediol, fluoroaliphatic polymeric ester, polyether-polysiloxane copolymer, and the like.

Examples of cationic surfactants useful for making cationic micelles include alkylamine salts, quaternary ammonium salts, sulphonium salts, and phosphonium 13 salts. Non-limiting examples of cationic surfactants include: the quaternary ammonium surfactants, which can have up to 26 carbon atoms include: alkoxylate quaternary ammonium (AQA) surfactants as discussed in U.S. Pat. No. 6,136,769; dimethyl hydroxyethyl quaternary ammonium as discussed in U.S. Pat. No. 6,004,922; dimethyl hydroxyethyl lauryl ammonium chloride; polyamine cationic surfactants as discussed in WO 98/35002, WO 98/35003, WO 98/35004, WO 98/35005, and WO 98/35006; cationic ester surfactants as discussed in U.S. Pat. Nos. 4,228,042, 4,239,660 4,260,529 and U.S. Pat. No. 6,022,844; and amino surfactants as discussed in U.S. Pat. No. 6,221,825 and WO 00/47708, specifically amido propyldimethyl amine (APA).

In some aspects of disclosure, the polycation includes a polyacrylate having one or more pendant amino groups. For example, the backbone of the polycation can be derived from the polymerization of acrylate monomers including, but not limited to, acrylates, methacrylates, acrylamides, and the like. In some aspects of disclosure, the polycation backbone is derived from polyacrylamide. In other aspects of disclosure, the polycation is a block copolymer, where segments or portions of the copolymer possess cationic groups or neutral groups depending upon the selection of the monomers used to produce the copolymer. In some aspects of disclosure, the pendant amino groups of polyacrylate is quaternary ammoniums. Accordingly, the polycation can be poly(acryloxyethyltrimethylammonium halide), poly(methacryloxyethyltrimethylammonium halide), poly(methacryloxy-2-hydroxypropyltrimethylammonium halide), or a co-polymer thereof.

The polyanion can be a synthetic polymer or naturally-occurring. Examples of other naturally-occurring polyanions include glycosaminoglycans such as condroitin sulfate, heparin, heparin sulfate, dermatan sulfate, and hyaluronic acid. In other embodiments, the acidic proteins having a net negative charge at neutral pH or proteins with a low pl can be used as naturally-occurring polyanions described herein. The anionic groups can be pendant to the polymer backbone and/or incorporated in the polymer backbone.

When the polyanion is a synthetic polymer, it is generally any polymer possessing anionic groups or groups that can be readily converted to anionic groups, for example, by adjusting the pH. Examples of groups that can be converted to anionic groups include, but are not limited to, carboxylate, sulfonate, phosphonate, boronate, sulfate, borate, or phosphate. Any cationic counterions can be used in association with the anionic polymers if the considerations discussed above are met.

In some aspects of disclosure, the polyanion is a polyphosphate. In other embodiments, the polyanion is a polyphosphate compound having from 5 to 90 mole % phosphate groups. For example, the polyphosphate can be a naturally-occurring compound such as, for example, highly phosphorylated proteins like phosvitin (an egg protein), dentin (a natural tooth phosphoprotein), casein (a phosphorylated milk protein), or bone proteins (*e.g.*, osteopontin).

Alternatively, the polyphosphoserine can be a synthetic polypeptide made by polymerizing the amino acid serine and then chemically phosphorylating the polypeptide. In other embodiments, the polyphosphoserine is produced by the polymerization of phosphoserine. In some embodiments, the polyphosphate is produced by chemically or enzymatically phosphorylating a protein (*e.g*., natural serine- or threonine-rich proteins). In further aspects of disclosure, the polyphosphate is produced by chemically phosphorylating a polyalcohol including, but not limited to, polysaccharides such as cellulose or dextran.

In other aspects of disclosure, the polyphosphate is a synthetic compound. For example, the polyphosphate can be a polymer with pendant phosphate groups attached to the polymer backbone and/or present in the polymer backbone, (*e.g*., a phosphodiester backbone).

In other aspects of disclosure, the polyanion is a micelle or mixed micelle formed with anionic surfactants. The anionic surfactant can be mixed with any of the nonionic surfactants described herein to create micelles with variable charge ratios. The micelles are polyanionic by virtue of the hydrophobic interactions that form a polyvalent micelle.

Other useful anionic surfactants include, but are not limited to, alkali metal and (alkyl)ammonium salts of: 1) alkyl sulfates and sulfonates such as sodium dodecyl sulfate, sodium 2-ethylhexyl sulfate, and potassium dodecanesulfonate; 2) sulfates of polyethoxylated derivatives of straight or branched chain aliphatic alcohols and carboxylic acids; 3) alkylbenzene or alkylnaphthalene sulfonates and sulfates such as sodium laurylbenzene-4-sulfonate and ethoxylated and polyethoxylated alkyl and aralkyl alcohol carboxylates; 5) glycinates such as alkyl sarcosinates and alkyl glycinates; 6) sulfosuccinates including dialkyl sulfosuccinates; 7) isothionate derivatives; 8) N-acyltaurine derivatives such as sodium N methyl-N-oleyltaurate); 9) amine oxides including alkyl and alkylamidoalkyldialkylamine oxides; and 10) alkyl phosphate mono or di-esters such as ethoxylated dodecyl alcohol phosphate ester, sodium salt.

Representative commercial examples of suitable anionic sulfonate surfactants include, for example, sodium lauryl sulfate, available as TEXAPON™ L-100 from Henkel Inc., Wilmington, Del., or as POLYSTEP™ B-3 from Stepan Chemical Co, Northfield, III.; sodium 25 lauryl ether sulfate, available as POLYSTEP™ B-12 from Stepan Chemical Co., Northfield, III.; ammonium lauryl sulfate, available as STANDAPOL™ A from Henkel Inc., Wilmington, Del.; and sodium dodecyl benzene sulfonate, available as SIPONATE™ DS-10 from Rhone-Poulenc, Inc., Cranbeny, N.J., dialkyl sulfosuccinates, having the tradename AEROSOL™ OT, commercially available from Cytec Industries, West Paterson, N.J.; sodium methyl taurate (available under the trade designation NIKKOL™ CMT30 from Nikko Chemicals Co., Tokyo, Japan); secondary alkane sulfonates such as Hostapur™ SAS which is a Sodium (C14-C17) secondary alkane sulfonates (alphaolefin sulfonates) available from Clariant Corp., Chariotte, N.C.; methyl-2-sulfoalkyl esters such as sodium methyl-2-sulfo(C₁₂₋₁₆)ester and disodium 2-sulfo(C₁₂-C₁₆) fatty acid available from Stepan Company under the trade designation ALPHASTE™ PC48; alkylsulfoacetates and alkylsulfosuccinates available as sodium laurylsulfoacetate (under the trade designation LANTHANOL™ LAL) and disodiumlaurethsulfosuccinate (STEPANMILD™ SL3), both from Stepan Company; alkylsulfates such as ammoniumlauryl sulfate commercially available under the trade 17 designation STEPANOL™ AM from Stepan Company, and or dodecylbenzenesulfonic acid sold under BIO-SOFT® AS-100 from Stepan Chemical Co. In some embodiments, the surfactant can be a disodium alpha olefin sulfonate, which contains a mixture of C₁₂ to C₁₆ sulfonates. In some embodiments, CALSOFT™ AOS-40 manufactured by Pilot Corp. can be used herein as the surfactant. In other embodiments, the surfactant is DOWFAX 2A1 or 2G manufactured by Dow Chemical, which are alkyl diphenyl oxide disulfonates.

Representative commercial examples of suitable anionic phosphate surfactants include a mixture of mono-, di- and tri-(alkyltetraglycolether)-o-phosphoric acid esters generally referred to as trilaureth-4-phosphate commercially available under the trade designation HOSTAPHAT™ 340KL from Clariant Corp., as well as PPG-5 cetyl 10 phosphate available under the trade designation CRODAPHOS™ SG from Croda Inc., Parsipanny, N.J.

Representative commercial examples of suitable anionic amine oxide surfactants those commercially available under the trade designations AMMONYX™ LO, LMDO, and CO, which are lauryldimethylamine oxide, laurylamidopropyldimethylaminc oxide, and cetyl amine oxide, all from Stepan Company.

In other aspects of disclosure, a phosphorous containing polymer, for example, a phospholipid, is converted into a polyanions. For example, a phospholipid or phosphosugar can be converted into a polyanion to produce a liposome or micelle. Thus, in this embodiment, the complex coacervate is a charged hydrophobically associated colloid.

The polyanion includes a polyacrylate having one or more pendant phosphate groups. For example, the polyanion can be derived from the polymerization of acrylate monomers including, but not limited to, acrylates, methacrylates, and the like. In other aspects of disclosure, the polyanion is a block co-polymer, where segments or portions of the co-polymer possess anionic groups and neutral groups depending upon the selection of the monomers used to produce the co-polymer.

In other aspects of the present disclosure, the polyanion is a polymer having at least one fragment having Formula VI: 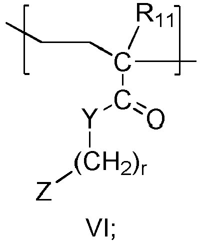 wherein
R₁₁ at each occurrence independently is hydrogen or an alkyl group;
r at each occurrence independently is an integer in the range of 1 and 10;
Y at each occurrence independently is oxygen, sulfur, or NR₁₂, wherein R₁₂ is hydrogen, an alkyl group, or an aryl group;
Z at each occurrence independently is an anionic group or a group that can be converted to an anionic group;
or a salt thereof.

In some aspects of the present disclosure, the salt is a pharmaceutically acceptable salt.

In various aspects of the present disclosure, Z is sulfate, sulfonate, carboxylate, borate, boronate, a substituted or unsubstituted phosphate, or a phosphonate.

In some aspects of disclosure, the polyanion is a polymer having at least one fragment having Formula VII: 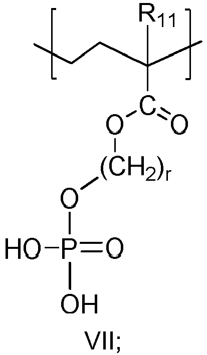 wherein
R₁₁ at each occurrence independently is hydrogen or an alkyl group, and
r at each occurrence independently is an integer from 1 to 10;
or a salt thereof.

In some aspects of disclosure, the salt is a pharmaceutically acceptable salt.

In certain aspects of disclosure, at least one R₁₁ is methyl and at least one r is 2. In some aspects of disclosure, the polyanion is the copolymerization product of methacryloxyethyl phosphate and acrylamide, where the mass average molecular weight is from 10,000 to 200,000, preferably 50,000, and has phosphate groups in the amount of 20 to 90 mol%.

The polycations and/or polyanions contain groups that permit crosslinking (or "curing") between the two polymers ("crosslinkable groups"). For example, a polycation of the present teachings can have one or more crosslinkable groups and/or a polyanion of the present teachings can have one or more crosslinkable groups. The mechanism of crosslinking can vary depending upon the selection of the crosslinking groups. In some embodiments, the crosslinking groups are electrophiles and nucleophiles. For example, the polyanion can have one or more electrophilic groups, and the polycations can have one or more nucleophilic groups capable of reacting with the electrophilic groups to produce new covalent bonds. Examples of electrophilic groups include, but are not limited to, anhydride groups, esters, ketones, lactams (*e.g.*, maleimides and succinimides), lactones, epoxide groups, isocyanate groups, and aldehydes. Examples of nucleophilic groups are presented herein. In some embodiments, the polycation and polyanion crosslink with one another via a Michael addition. For example, the polycation can have one or more nucleophilic groups such as, for example, a hydroxyl or thiol group that can react with an olefinic group present on the polyanion.

In some embodiments, the crosslinkable group on the polyanion comprises an olefinic group and the crosslinkable group on the polycation comprises a nucleophilic group that reacts with the olefinic group to produce a new covalent bond. In other embodiments, the crosslinkable group on the polycation comprises an olefinic group and the crosslinkable group on the polyanion comprises a nucleophilic group that reacts with the olefinic group to produce a new covalent bond.

In other embodiments, the polycation and polyanion each have a crosslinkable group, for example, an actinically crosslinkable group. As used herein, "actinically crosslinkable group" in reference to curing or polymerizing means that the crosslinking between the polycation and polyanion is performed by actinic irradiation, such as, for example, UV irradiation, visible light irradiation, ionized radiation (*e.g*., gamma ray or X-ray irradiation), microwave irradiation, and the like. Actinic curing methods are well-known to a person skilled in the art. The actinically crosslinkable group can be an unsaturated organic group such as, for example, an olefinic group. Examples of olefinic groups useful herein include, but are not limited to, an acrylate group, a methacrylate group, an acrylamide group, a methacrylamide group, an allyl group, a vinyl group, a vinylester group, or a styrenyl group. In other embodiments, the actinically crosslinkable group is an azido group. For example, crosslinking can occur between the polycation and polyanion via light activated crosslinking through azido groups.

Any of the polymers described above (synthetic or naturally-occurring) that can be used as the polycation and polyanion can be modified to include an actinically crosslinkable group discussed herein. For example, a polyphosphate can be modified to include the actinically crosslinkable group(s).

In some aspects of the present disclosure, the polycation and/or polyanion can include at least one fragment having Formula VIII: 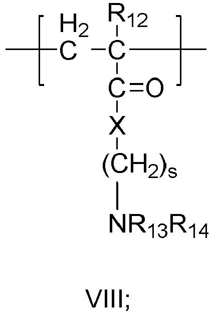 wherein
R₁₂, R₁₃, and R₁₄ at each occurrence independently are hydrogen, alkyl, alkenyl, alkynyl, aryl, arylalkyl, or alkoxy;
X is oxygen or NR₁₅, where R₁₅ is hydrogen or an alkyl group; and
s is an integer in the range of 1 and 10,
or a salt thereof, wherein at least one of R₁₃ or R₁₄ is a crosslinkable group.

In some aspects of the present disclosure, referring to Formula VIII, R₁₂ is methyl, R₁₃ is hydrogen, R₁₄ is an acrylate or methacrylate group, X is NH, and s is 2. In some aspects of the present disclosure, the crosslinkable group is an actinically crosslinkable group.

In some aspects of disclosure, the polycation is a polyamino compound modified to include one or more acrylate or methacrylate groups. Any of the polyamino compounds described herein that is useful as a polycation of the present teachings can be chemically modified to incorporate one or more acrylate or methacrylate groups. An example of this is where the branched polyamino compound has methacrylate groups attached to each arm of the polyamine. The number of acrylate or methacrylate groups attached to the polyamino compound can vary as needed.

In some embodiments, the polyanion is a phosphate compound modified to include one or more acrylate or methacrylate groups. Any of the phosphate compounds described herein that is useful as a polyanion of the present teachings can be chemically modified to incorporate one or more acrylate or methacrylate groups. An example is where a phosphate compound with a pendant carboxylic acid group was reacted with glycidyl methacrylate to produce the phosphate compound with a terminal methacrylate group. The number of acrylate or methacrylate groups attached to the phosphate compound can vary as needed.

In other embodiments, the crosslinkable group includes a dihydroxy-substituted aromatic group capable of undergoing oxidation in the presence of an oxidant. In some embodiments, the dihydroxy-substituted aromatic group is an ortho-dihydroxy aromatic group capable of being oxidized to the corresponding quinone. In other embodiments, the dihydroxyl-substituted aromatic group is a dihydroxyphenol or halogenated dihydroxyphenol group such as, for example, DOPA and catechol (3,4-dihydroxyphenol). For example, in the case of DOPA, it can be oxidized to dopaquinone. Dopaquinone is capable of either reacting with a neighboring DOPA group or another nucleophilic group. In the presence of an oxidant such as oxygen or other additives including, but not limited to, peroxides, periodates (*e.g*., NalO₄), persulfates, permanganates, dichromates, transition metal oxidants (*e.g*., a Fe⁺³ compound, osmium tetroxide), or enzymes (*e.g.*, catechol oxidase), the dihydroxyl-substituted aromatic group can be oxidized.

In some embodiments, a polyanion of the present teachings is the polymerization product between two or more monomers, where one of the monomers has a dihydroxy aromatic group covalently attached to the monomer. For example, the polyanion can be the polymerization product between (1) a phosphate acrylate and/or phosphate methacrylate and (2) a second acrylate and/or second methacrylate having a dihydroxy aromatic group covalently bonded to the second acrylate or second methacrylate. In other embodiments, the polyanion is the polymerization product between methacryloxyethyl phosphate and dopamine methacrylamide. In each of these polymers, an acrylate containing the pendant ortho-dihydroxyphenyl residue is polymerized with the appropriate monomers to produce the polyanion with pendant ortho-dihydroxyphenyl residues. Oxidation of ortho-dihydroxyphenyl groups results in orthoquinone groups, a reactive intermediate that can crosslink (*i.e.,* react) with nucleophiles such as, for example, amino, hydroxyl, or thiol groups via a Michael-type addition to form a new covalent bond. For example, a lysyl group on the polycation can react with the orthoquinone residue on the polyanion to produce new covalent bonds. Although an ortho-dihydroxyphenyl group is a suitable crosslinkable group, other groups such as, for example, tyrosine can be used herein. The importance of crosslinking with respect to the use of the complex coacervates described herein will be discussed below.

In certain embodiments, the oxidant used above is stabilized. For example, a compound that forms a complex with periodate that is not redox active can result in a stabilized oxidant. In other words, the periodate is stabilized in a non-oxidative form and cannot oxidize the ortho-dihydroxy-substituted aromatic group while in the complex. The complex is reversible: there is a small amount of uncomplexed periodate formed. Without wishing to be bound by any particular theory, an ortho-dihydroxyl-substituted aromatic group in a polycation or polyanion of the present teachings competes with the compound for the small amount of free periodate. As the free periodate is oxidized, more is released from the equilibrium complex. In some embodiments, sugars possessing a cis,cis-1,2,3-triol grouping on a six-membered ring form competitive periodate complexes. An example of a specific compound that forms stable periodate complex is 1,2-O-isopropylidene-alpha-D-glucofuranose (A. S. Perlin and E. von Rudloff, Canadian Journal of Chemistry. Volume 43 (1965)). The stabilized oxidant can control the rate of crosslinking. Without wishing to be bound by any particular theory, the stabilized oxidant can control the rate of oxidation providing time to add the oxidant and position the substrate before the adhesive hardens irreversibly.

In other embodiments, the crosslinkable groups present on the polycation and/or polyanion form coordination complexes with transition metal ions. For example, a transition metal ion can be added to a mixture of polycation and polyanion, where both polymers contain groups capable of coordinating transition metal ions. Examples of coordinating sidechains are catechols, imidazoles, phosphates, carboxylic acids, and combinations. The rate of coordination and dissociation can be controlled by the selection of the coordination group, the transition metal ion, and the pH. Thus, in addition to covalent crosslinking as described above, crosslinking can occur through electrostatic, ionic, coordinative, or other non-covalent bonding. Transition metal ions such as, for example, iron, copper, vanadium, zinc, and nickel can be used herein.

In various embodiments, the complex coacervate includes a multivalent crosslinker. In some embodiments, the multivalent crosslinker has two or more nucleophilic groups (*e.g.*, hydroxyl, thiol, etc.) that react with crosslinkable groups (*e.g.*, olefinic groups) present on the polycation and polyanion via a Michael addition reaction to produce a new covalent bond. In some embodiments, the multivalent crosslinker is a dithiol or trithiol compound.

The coacervates described herein can include a reinforcing component. The term "reinforcing component" is defined herein as any component that enhances or improves the mechanical properties (*e.g.*, cohesiveness, fracture toughness, elastic modulus, the ability to release and bioactive agents, dimensional stability after curing, etc.) of the complex coacervate prior to or after curing of the coacervate when compared to the same coacervate that does not include the reinforcing component. The mode in which the reinforcing component can enhance the mechanical properties of the coacervate can vary, and will depend upon the intended application of the adhesives as well as the selection of the polycation, polyanion, and reinforcing component. For example, upon curing the coacervate, the polycations and/or polyanions present in the coacervate can covalently crosslink with the reinforcing component. In other embodiments, the reinforcing component can occupy a space or "phase" in the coacervate, which ultimately increases the mechanical properties of the coacervate. Examples of reinforcing components useful herein are provided below.

In some embodiments, the reinforcing component is a polymerizable monomer. The polymerizable monomer entrapped in the complex coacervate can be any water soluble monomer capable of undergoing polymerization in order to produce an interpenetrating polymer network. The selection of the polymerizable monomer can vary depending upon the application. Factors such as molecular weight can be altered to modify the solubility properties of the polymerizable monomer in water as well as the mechanical properties of the resulting coacervate.

The selection of the functional group on the polymerizable monomer determines the mode of polymerization. For example, the polymerizable monomer can be a polymerizable olefinic monomer that can undergo polymerization through mechanisms such as, for example, free radical polymerization and Michael addition reactions. In some embodiments, the polymerizable monomer has two or more olefinic groups. In some embodiments, the monomer comprises one or two actinically crosslinkable groups. As discussed above, "actinically crosslinkable group" in reference to curing or polymerizing means that the crosslinking between the polymerizable monomer is performed by actinic irradiation, such as, for example, UV irradiation, visible light irradiation, ionized radiation (*e.g.,* gamma ray or X-ray irradiation), microwave irradiation, and the like. This can be performed in the presence of a photoinitiator, which is discussed in detail herein. Actinic curing methods are well-known to a person skilled in the art. Examples of actinically crosslinkable group useful herein include, but are not limited to, a pendant acrylate group, methacrylate group, acrylamide group, methacrylamide group, allyl, vinyl group, vinylester group, or styrenyl group. Alternatively, polymerization can be performed in the presence of an initiator and coinitiator which are also discussed in detail below.

Examples of water-soluble polymerizable monomers include, but are not limited to, hydroxyalkyl methacrylate (HEMA), hydroxyalkyl acrylate, N-vinyl pyrrolidone, N-methyl-3-methylidene-pyrrolidone, allyl alcohol, N-vinyl alkylamide, N-vinyl-N-alkylamide, acrylamides, methacrylamide, (lower alkyl)acrylamides and methacrylamides, and hydroxyl-substituted (lower alkyl)acrylamides and hydroxyl-substituted methacrylamides. In some embodiments, the polymerizable monomer is a diacrylate compound or dimethacrylate compound. In other embodiments, the polymerizable monomer is a polyalkylene oxide glycol diacrylate or dimethacrylate. For example, the polyalkylene can be a polymer of ethylene glycol, propylene glycol, or block co-polymers thereof. In some embodiments, the polymerizable monomer is polyethylene glycol diacrylate or polyethylene glycol dimethacrylate. In some embodiments, the polyethylene glycol diacrylate or polyethylene glycol dimethacrylate has a Mₙ of 200 to 2,000, 400 to 1,500, 500 to 1,000, 500 to 750, or 500 to 600.

In other embodiments, the reinforcing component is a nanostructure. Depending upon the selection of the nanostructure, the polycation and/or polyanion can be covalently crosslinked to the nanostructure. Alternatively, the nanostructures can be physically entrapped within the coacervate. Nanostructures can include, for example, nanotubes, nanowires, nanorods, or a combination thereof. In the case of nanotubes, nanowires, and nanorods, one of the dimensions of the nanostructure is less than 100 nm.

The nanostructures useful herein can be composed of organic and/or inorganic materials. In some embodiments, the nanostructures are composed of organic materials like carbon or inorganic materials including, but not limited to, boron, molybdenum, tungsten, silicon, titanium, copper, bismuth, tungsten carbide, aluminum oxide, titanium dioxide, molybdenum disulphide, silicon carbide, titanium diboride, boron nitride, dysprosium oxide, iron (III) oxide-hydroxide, iron oxide, manganese oxide, titanium dioxide, boron carbide, aluminum nitride, or any combination thereof.

In certain embodiments, the nanostructures are functionalized in order to react (*i.e.,* crosslink) with the polycation and/or polyanion. For example, carbon nanotubes can be functionalized with -OH or-COOH groups. In other embodiments, it is desirable to use two or more different types of nanostructures. For example, a carbon nanostructure can be used in combination with one or more inorganic nanostructures.

In other embodiments, the reinforcing component is a water-insoluble filler. The filler can have a variety of different sizes and shapes, ranging from particles to fibrous materials. In some embodiments, the filler is a nano-sized particle. Without wishing to be bound by any particular theory, compared to micron-sized silica fillers, nanoscale fillers can have several desirable properties. First, the higher specific surface area of nano- vs. microparticles can increase the stress transfer from the polymer matrix to the rigid filler. Second, smaller volumes of nanofiller are required than of the larger micron-sized particles for a greater increase in toughness. Additionally, the smaller diameters and lower fill volumes of nanoparticles can reduce viscosity of the uncured adhesive, which can have direct benefits for processability. This is advantageous, as the complex coacervate can retain its injectable character while potentially increasing bond strengths dramatically. Third, maximum toughening requires uniform dispersion of the filler particles within the complex coacervate. Nanoscale colloidal particles, again because of the small diameter, lend themselves more readily to stable dispersions within the complex coacervate.

Without wishing to be bound by any particular theory, compared to micron-sized silica fillers, nanoscale fillers can have several desirable properties. First, the higher specific surface area of nano- vs. microparticles can increase the stress transfer from the polymer matrix to the rigid filler. Second, smaller volumes of nanofiller are required than of the larger micron-sized particles for a greater increase in toughness. Additionally, the smaller diameters and lower fill volumes of nanoparticles can reduce viscosity of the uncured adhesive, which can have direct benefits for processability. This is advantageous, as the coacervate can retain its injectable character while potentially increasing bond strengths dramatically. Third, maximum toughening requires uniform dispersion of the filler particles within the coacervate. Nanoscale colloidal particles, again because of the small diameter, lend themselves more readily to stable dispersions within the coacervate.

In some embodiments, the filler comprises a metal oxide, a ceramic particle, or a water insoluble inorganic salt. Examples of the nanoparticles or nanopowders useful herein include: Ag, 99.95%, 100 nm; Ag, 99.95%, 20-30 nm; Ag, 99.95%, 20-30 nm, PVP coated; Ag, 99.9%, 50-60 nm; Ag, 99.99%, 30-50 nm, oleic acid coated; Ag, 99.99%, 15 nm, 10wt%, self-dispersible; Ag, 99.99%, 15 nm, 25wt%, self-dispersible; Al, 99.9%, 18nm; Al, 99.9%, 40-60 nm; Al, 99.9%, 60-80 nm; Al, 99.9%, 40-60 nm, low oxygen; Au, 99.9%, 100 nm; Au, 99.99%, 15 nm, 10wt%, self-dispersible; B, 99.9999%; B, 99.999%; B, 99.99%; B, 99.9%; B, 99.9%, 80 nm; Diamond, 95%, 3-4 nm; Diamond, 93%, 3-4 nm; Diamond, 55-75 %, 4-15 nm; Graphite, 93%, 3-4 nm; Super Activated Carbon, 100 nm; Co, 99.8%, 25-30 nm; Cr, 99.9%, 60-80 nm; Cu, 99.5%, 300 nm; Cu, 99.5%, 500 nm; Cu, 99.9%, 25 nm; Cu, 99.9%, 40-60 nm; Cu, 99.9%, 60-80 nm; Cu, 5-7 nm, dispersion, oil soluble; Fe, 99.9%, 20 nm; Fe, 99.9%, 40-60 nm; Fe, 99.9%, 60-80 nm; Carbonyl-Fe, micro-sized; Mo, 99.9%, 60-80 nm; Mo, 99.9%, 0.5-0.8 □m; Ni, 99.9%, 500 nm (adjustable); Ni, 99.9%, 20 nm; Ni coated with carbon, 99.9%, 20 nm; Ni, 99.9%, 40-60 nm; Ni, 99.9%, 60-80 nm; Carbonyl-Ni, 2-3 □m; Carbonyl-Ni, 4-7 □m; Carbonyl-Ni-AI (Ni Shell, Al Core); Carbonyl-Ni-Fe Alloy; Pt, 99.95%, 5 nm, 10wt%, self-dispersible; Si, Cubic, 99%, 50 nm; Si, Polycrystalline, 99.99995%, lumps; Sn, 99.9%, <100 nm; Ta, 99.9%. 60-80 nm; Ti, 99.9%. 40-60 nm; Ti, 99.9%, 60-80 nm; W, 99.9%, 40-60 nm; W, 99.9%, 80-100 nm; Zn, 99.9%, 40-60 nm; Zn, 99.9%, 80-100 nm; AIOOH, 10-20nm, 99.99%; Al₂O₃ alpha, 98+%, 40 nm; Al₂O₃ alpha, 99.999%, 0.5-10 □m; Al₂O₃ alpha, 99.99%, 50 nm; Al₂O₃ alpha, 99.99%, 0.3-0.8 □m; Al₂O₃ alpha, 99.99%, 0.8-1.5 □m; Al₂O₃ alpha, 99.99%, 1.5-3.5 □m; Al₂O₃ alpha, 99.99%, 3.5-15 □m; Al₂O₃ gamma, 99.9%, 5 nm; Al₂O₃ gamma, 99.99%, 20 nm; Al₂O₃ gamma, 99.99%, 0.4-1.5 □m; Al₂O₃ gamma, 99.99%, 3-10 □m; Al₂O₃ gamma, extrudate; Al₂O₃ gamma, extrudate; Al(OH)₃, 99.99%, 30-100 nm; Al(OH)₃, 99.99%, 2-10 □m; Aluminium Iso-Propoxide (AIP), C₉H₂₁O₃Al, 99.9%; AIN, 99%, 40 nm; BaTiO₃, 99.9%, 100 nm; BBr₃, 99.9%; B₂O₃, 99.5%, 80 nm; BN, 99.99%, 3-4 □m; BN, 99.9%, 3-4 □m; B₄C, 99%, 50 nm; Bi₂O₃, 99.9%, <200 nm; CaCO₃, 97.5%, 15-40 nm; CaCO₃, 15-40 nm; Ca₃(PO₄)₂, 20-40 nm; Ca₁₀(PO₄)₆(OH)₂, 98.5%, 40 nm; CeO₂, 99.9%, 10-30 nm; CoO,<100 nm; CO₂O₃, <100 nm; CO₃O₄, 50 nm; CuO, 99+%, 40 nm; Er₂O₃, 99.9%, 40-50 nm; Fe₂O₃ alpha, 99%, 20-40 nm; Fe₂O₃ gamma, 99%, 20-40 nm; Fe₃O₄, 98+%, 20-30 nm; Fe₃O₄, 98+%, 10-20 nm; Gd₂O₃, 99.9%<100 nm; HfO₂, 9 9.9%, 100 nm; In₂O₃:SnO₂=90:10, 20-70 nm; In₂O₃, 99.99%, 20-70 nm; In(OH)₃, 99.99%, 20-70 nm; LaB₆, 99.0%, 50-80 nm; La₂O₃, 99.99%, 100 nm; LiFePO₄, 40 nm; MgO, 99.9%, 10-30 nm; MgO, 99%, 20 nm; MgO, 99.9%, 10-30 nm; Mg(OH)₂, 99.8%, 50 nm; Mn₂O₃, 98+%, 40-60 nm; MoCl₅, 99.0%; Nd₂O₃, 99.9%, <100 nm; NiO, <100 nm; Ni₂O₃, <100 nm; Sb₂O₃, 99.9%, 150 nm; SiO₂, 99.9%, 20-60 nm; SiO₂, 99%, 10-30 nm, treated with silane coupling agents; SiO₂, 99%, 10-30 nm, treated with Hexamethyldisilazane; SiO₂, 99%, 10-30 nm, treated with Titanium Ester; SiO₂, 99%, 10-30 nm, treated with silanes; SiO₂, 10-20 nm, modified with amino group, dispersible; SiO₂, 10-20 nm, modified with epoxy group, dispersible; SiO₂, 10-20 nm, modified with double bond, dispersible; SiO₂, 10-20 nm, surface modified with double layer, dispersible; SiO₂, 10-20 nm, surface modified, super-hydrophobic and oleophilic, dispersible; SiO₂, 99.8%, 5-15 nm, surface modified, hydrophobic and oleophilic, dispersible; SiO₂, 99.8%, 10-25 nm, surface modified, super-hydrophobic, dispersible; SiC, beta, 99%, 40 nm; SiC, beta, whisker, 99,9%; Si₃N₄, amorphous, 99%, 20 nm; Si₃N₄ alpha, 97.5-99%, fiber, 100nmX800 nm;
SnO₂, 99.9%, 50-70 nm; ATO, SnO₂:Sb₂O₃=90:10, 40 nm; TiO₂ anatase, 99.5%, 5-10 nm; TiO₂ Rutile, 99.5%, 10-30 nm; TiO₂ Rutile, 99%, 20-40 nm, coated with SiO₂, highly hydrophobic; TiO₂ Rutile, 99%, 20-40 nm, coated with SiO₂/Al₂O₃; TiO₂ Rutile, 99%, 20-40 nm, coated with Al₂O₃, hydrophilic; TiO₂ Rutile, 99%, 20-40 nm, coated with SiO₂/Al₂O₃/Stearic Acid; TiO₂ Rutile, 99%, 20-40 nm, coated with Silicone Oil, hydrophobic; TiC, 99%, 40 nm; TiN, 97+%, 20 nm; WO₃, 99.5%,<100 nm; WS₂, 99.9%, 0.8 nm; WCl₆, 99.0%; Y₂O₃, 99.995%, 30-50 nm; ZnO, 99.8%, 10-30 nm; ZnO, 99%, 10-30 nm, treated with silane coupling agents; ZnO, 99%, 10-30 nm, treated with stearic acid; ZnO, 99%, 10-30 nm, treated with silicone oil; ZnO, 99.8%, 200 nm; ZrO₂, 99.9%, 100 nm; ZrO₂, 99.9%, 20-30 nm; ZrO₂-3Y, 99.9%, 0.3-0.5 □m; ZrO₂-3Y, 25 nm; ZrO₂-5Y, 20-30 nm; ZrO₂-8Y, 99.9%, 0.3-0.5 □m; and ZrO₂-8Y, 20 nm; ZrC, 97+%, 60 nm.

In some embodiments, the filler is nanosilica. Nanosilica is commercially available from multiple sources in a broad size range. For example, aqueous Nexsil colloidal silica is available in diameters from 6-85 nm from Nyacol Nanotechnologies, Inc. Amino-modified nanosilica is also commercially available, from Sigma Aldrich for example, but in a narrower range of diameters than unmodified silica. Nanosilica does not contribute to the opacity of the coacervate, which is an important attribute of the adhesives and glues produced therefrom.

In other embodiments, the filler can be composed of calcium phosphate. In some embodiments, the filler can be hydroxyapatite, which has the formula Ca₅(PO₄)₃OH. In other embodiments, the filler can be a substituted hydroxyapatite. A substituted hydroxyapatite is hydroxyapatite with one or more atoms substituted with another atom. The substituted hydroxyapatite is depicted by the formula M₅X₃Y, where M is Ca, Mg, Na; X is PO₄ or CO₃; and Y is OH, F, CI, or CO₃. Minor impurities in the hydroxyapatite structure may also be present from the following ions: Zn, Sr, Al, Pb, Ba.

In other embodiments, the calcium phosphate comprises a calcium orthophosphate. Examples of calcium orthophosphates include, but are not limited to, monocalcium phosphate anhydrate, monocalcium phosphate monohydrate, dicalcium phosphate dihydrate, dicalcium phosphate anhydrous, octacalcium phosphate, beta tricalcium phosphate, alpha tricalcium phosphate, super alpha tricalcium phosphate, tetracalcium phosphate, amorphous tricalcium phosphate, or any combination thereof. In other embodiments, the calcium phosphate also includes calcium-deficient hydroxyapalite, which can preferentially adsorb bone matrix proteins.

In certain embodiments, the filler is functionalized with one or more polymerizable functional groups that are capable of capable of reacting with a crosslinkable group on the polycation and/or polyanion and, when present the polymerizable monomer. In this embodiment, the filler is covalently attached to the polycation and/or polyanion and, when present, the interpenetrating network. For example, aminated silica can be reacted with a compound that possesses (1) a functional group capable of reacting with the amino groups present on the silica and (2) an olefinic group capable of undergoing polymerization. Thus, the olefinic groups are covalently attached to the silica. In some embodiments, aminated nanosilica is reacted with acryloyl chloride to covalently attach an acrylate group to the silica. Depending upon the selection of the polycation and polyanion, the filler can react with these components to covalently attach to the complex coacervate and, when present, interpenetrating network.

In other embodiments, the filler includes one or more nucleophilic groups capable of reacting with a crosslinkable group on the polycation and/or polyanion and, when present, the polymerizable monomer. For example, the filler particle can be modified with surface amines or thiols (*i.e.,* nucleophiles) that can react with electrophiles (*e.g.*, ortho-quinones produced by the oxidizing o-dihydroxyphenyl groups) in the coacervate polymer network. In other embodiments, nucleophilic groups present on the filler react with olefinic groups present in the polymerizable monomer and/or coacervate polymer network via a Michael addition reaction.

In other embodiments, the filler is modified to produce charged groups such that the filler can form electrostatic bonds with the coacervate polymer network and/or the interpenetrating network when a polymerizable monomer is used. For example, aminated silica can be added to a solution and the pH adjusted so that the amino groups are protonated and available for electrostatic bonding.

In some embodiments, the reinforcing component is micelles or liposomes. In general, the micelles and liposomes used in this embodiment are different from the micelles or liposomes used as polycations and polyanions for preparing the coacervate. The micelles and liposomes can be prepared from the nonionic, cationic, or anionic surfactants described above. The charge of the micelles and liposomes can vary depending upon the selection of the polycation or polyanion as well as the intended use of the coacervate. In some embodiments, the micelles and liposomes are used to solubilize hydrophobic compounds such pharmaceutical compounds. Thus, in addition to being used as adhesives, the reinforced complex coacervates described herein can be effective as a bioactive delivery device.

In certain embodiments, the coacervate also includes one or more initiators entrapped in the coacervate. Examples of initiators useful herein include a thermal initiator, a chemical initiator, or a photoinitiator. In some embodiments, when the coacervate includes a polymerizable monomer as the reinforcing component, when the initiator is activated, polymerization of the polymerizable monomer entrapped in the coacervate occurs to produce the interpenetrating network. Additionally, crosslinking can occur between the polycation and polyanion, as well as with the interpenetrating network.

Examples of photoinitiators include, but are not limited to, a phosphine oxide, a peroxide group, an azide group, an □-hydroxyketone, or an □-aminoketone. In some embodiments, the photoinitiator includes, but is not limited to, camphorquinone, benzoin methyl ether, I-hydroxycyclohexylphenyl ketone, or Darocure® or Irgacure® types, for example Darocure® 1173 or Irgacure® 2959. The photoinitiators disclosed in European Patent No. 0632329, which are incorporated by reference, can be used herein. In other embodiments, the photoinitiator is a water-soluble photoinitiator including, but not limited to, riboflavin, eosin, eosin y, and rose Bengal.

In some embodiments, the initiator has a positively charged functional group. Examples include 2,2'-azobis[2-(5-methyl-2-imidazolin-2-yl)propane]-dihydrochloride; 2,2'-azobis[2-(2-imidazolin-2-yl) propane]dihydrochloride; 2,2-azobis[2-(2-imidazo-lin-2-yl)propane]disulfate dehydrate; 2,2'-azobis(2-methylpropionamidine)dihydrochloride; 2,2'-azobis[2-(3,4,5,6-tetrahydropyrimidin-2-yl)propane]dihydrochloride; azobis(2-[1-(2-hydroxyethyl)-2-imidazolin-2-yl]propane)dihydrochloride; 2,2'-azobis(1-imino-1-pyrrolidino-2-ethylpropane)dihydrochloride and combinations thereof.

In other embodiments, the initiator is an oil soluble initiator. In some embodiments, the oil soluble initiator includes organic peroxides or azo compounds. Examples of organic peroxides include ketone peroxides, peroxyketals, hydroperoxides, dialkyl peroxides, diacyl peroxides, peroxydicarbonates, peroxyesters, and the like. Some specific non-limiting examples of organic peroxides that can be used as the oil soluble initiator include: lauroyl peroxide, 1,1-bis(t-hexylperoxy)-3,3,5-trimethylcyclohexane, 1,1-bis(t-butylperoxy)-3,3,5-trimethylcyclohexane, t-butylperoxylaurate, t-butylperoxyisopropylmonocarbonate, t-butylperoxy-2-ethylhexylcarbonate, di-t-bulylperoxyhexahydro-terephthalate, dicumyl peroxide, 2,5-dimethyl-2,5-di(t-butylperoxy)hexane, di-t-butyl peroxide, t-butylperoxy-2-ethylhexanoate, bis(4-t-butylcyclohexyl)peroxydi-carbonate, t-amylperoxy-3,5,5-trimethylhexanoate, 1,1-di(t-amylperoxy)-3,3,5-trimethylcyclohexane, benzoyl-peroxide, t-butylperoxyacetate, and the like.

Some specific non-limiting examples of azo compounds that can be used as the oil soluble initiator include: 2,2'-azobis-isobutyronitrile, 2,2'-azobis-2,4-dimethylvaleronitrile, 1,1'-azobis-I-cyclohexane-carbonitrile, dimethyl-2,2'-azobisisobutyrate, 1,1'-azobis-(1-acetoxy-1-phenylethane), 4,4'-azobis(4-cyanopentanoic acid) and its soluble salts (e.g., sodium, potassium), and the like.

In some embodiments, the initiator is a water-soluble initiator including, but not limited to, potassium persulfate, ammonium persulfate, sodium persulfate, and mixtures thereof. In other embodiments, the initiator is an oxidation-reduction initiator such as the reaction product of the above-mentioned persulfates and reducing agents such as sodium metabisulfite and sodium bisulfite; and 4,4'-azobis(4-cyanopentanoic acid) and its soluble salts (e.g., sodium, potassium).

In certain embodiments, multiple initiators are used to broaden the absorption profile of the initiator system in order to increase the initiation rate. For example, two different photoinitiators can be employed that are activated by different wavelengths of light. In other embodiments, a co-initiator is used in combination with any of the initiators described herein. In some embodiments, the co-initiator is 2-(diethylamino)ethyl acrylate, 2-(dimethylamino)ethyl acrylate, 2-(dimethylamino)ethyl benzoate, 2-(dimethylamino)ethyl methacrylate, 2-ethylhexyl-4-(dimethylamino)benzoate, 3-(dimethylamino)propyl acrylate, 4,4'-bis(diethylamino)benzophenone, or 4-(diethylamino)benzophenone.

In certain embodiments, the initiator and/or co-initiator are covalently attached to the polycation and/or polyanion. For example, the initiator and/or co-initiator can be copolymerized with monomers used to make the polycation and/or polyanion. In some embodiments, the initiators and co-initiators possess polymerizable olefinic groups such as acrylate and methacrylate groups (e.g., see examples of co-initiators above) that can be copolymerized with monomers described used to make the polycation and polyanion. In other embodiments, the initiators are chemically grafted onto the backbone of the polycation and polyanion. Thus, in these embodiments, the photoinitiator and/or co-initiator are covalently attached to the polymer and pendant to the polymer backbone. This approach will simply formulation and possibly enhance storage and stability.

In various embodiments, the complex coacervates include one or more multivalent cations (*i.e.,* cations having a charge of +2 or greater). In some embodiments, the multivalent cation is a divalent cation composed of one or more alkaline earth metals. For example, the divalent cation can be a mixture of Ca⁺² and Mg⁺². In other embodiments, transition metal ions with a charge of +2 or greater are used as the multivalent cation. The concentration of the multivalent cations can determine the rate and extent of coacervate formation. Without wishing to be bound by any particular theory, weak cohesive forces between particles in the fluid may be mediated by multivalent cations bridging excess negative surface charges. The amount of multivalent cation used herein can vary. In some embodiments, the amount is based upon the number of anionic groups and cationic groups present in the polyanion and polycation.

The synthesis of the complex coacervates described herein can be performed using a number of techniques and procedures. In some embodiments, an aqueous solution of polycation is mixed with an aqueous solution of polyanion, where one or both of the solutions contain the polymerizable monomer and other optional components (*e.g.,* fillers, initiators, etc.). In certain embodiments, the pH of each solution is adjusted to a desired pH (*e.g.,* physiological pH) prior lo mixing with one another to produce the complex coacervate. Alternatively, after mixing the polycation, polyanion, polymerizable monomer, and optional components, the pH of the resulting solution is adjusted to produce the complex coacervate. Upon mixing, the complex coacervate forms a fluid that settles to the bottom of the solution, at which time the supernatant is removed and the complex coacervate is ready for use.

In various embodiments, the complex coacervate is cured to induce crosslinking within the coacervate to produce a cured complex coacervate. The cured complex coacervate can be used as an adhesive. Depending upon the selection of starting materials, varying degrees of crosslinking can occur throughout the coacervate during curing. In some embodiments, the polycations and polyanions are crosslinked with one another by covalent bonds upon curing. In other embodiments, the polycations and/or polyanions are crosslinked with the reinforcing component.

In some embodiments, after the complex coacervate has been produced and applied to a substrate or adherend, it is converted to a load bearing adhesive bond using techniques known in the art. In some embodiments, the adhesive is produced by the process comprising
(a) providing an complex coacervate described herein comprising a polymerizable monomer, and
(b) curing the complex coacervate to polymerize the polymerizable monomer and produce an interpenetrating network.

In this embodiment, step (b) involves curing the complex coacervate in order to polymerize the polymerizable monomer and produce an interpenetrating network throughout the coacervate. In some embodiments, the polycations and polyanions are crosslinked with one another by covalent bonds upon curing. In other embodiments, the polycations and/or polyanions are crosslinked with the interpenetrating network. For example, the polymerizable monomer can possess groups that can covalently crosslink with the polycation and/or polyanion, which enhances the overall mechanical properties of the coacervate.

The method of polymerizing the polymerizable monomer to produce the interpenetrating network can vary depending upon the nature of the polymerizable monomer. For example, if the polymerizable monomer has one or more polymerizable olefinic groups, an initiator and a coinitiator can be incorporated into the coacervate using the method described above, and the coacervate can be exposed to light to produce the interpenetrating network. Any of the initiators and coinitiators described are can be used herein.

In certain embodiments, when the polycation and polyanion possess orthogonally crosslinkable groups, the groups are crosslinked with one another prior to the polymerization of the polymerizable monomer, after the polymerization of the polymerizable monomer, or simultaneously with the polymerization of the polymerizable monomer. For example, using the techniques described above and in the Examples, the coacervate can be contacted with an oxidant such as O₂, NalO₄, a peroxide, or a transition metal oxidant in order to facilitate crosslinking. As discussed above, the rate of oxidative crosslinking can be controlled when the oxidant is combined with certain sugars.

In various embodiments, the polycation and/or polyanion are covalently attached to the interpenetrating network. For example, the polycation and polyanion can include olefinic groups capable of polymerizing with the polymerizable monomer to form a covalent bond with the interpenetrating network. In other embodiments, the polycation and polyanion comprises nucleophilic groups (*e.g.,* thiols or amines) capable of reacting with groups on the interpenetrating network (*e.g.,* olefinic groups).

In other embodiments, when the reinforcing component is a filler, the filler is functionalized such that it forms covalent or non-covalent bonds with the polycation, polyanion, and/or interpenetrating network. For example, if the filler is functionalized with olefinic groups such as acrylate groups, it can polymerize with the polymerizable monomer such that the filler is covalently bonded to the resulting interpenetrating network. Alternatively, the filler can be modified with nucleophilic groups capable of reacting with electrophilic groups on the polycation, polyanion, and/or interpenetrating network. In other embodiments, the filler possesses groups that permit electrostatic interactions between the polycation, polyanion, interpenetrating network, or any combination thereof.

The interpenetrating polymer network is biodegradable and biocompatible, for example, for medical applications. Thus, the polymerizable monomer is selected such that a biodegradable and biocompatible interpenetrating polymer network is produced upon polymerization. For example, the polymerizable monomer can possess cleavable ester linkages. In some
embodiments, the polymerizable monomer is hydroxypropyl methacrylate (HPMA), which will produce a biocompatible interpenetrating network. In other embodiments, biodegradable moieties are used to polymerize biocompatible water soluble monomers such as, for example, alkyl methacrylamides. A part of the interpenetrating network can be enzymatically degradable, like a peptide or a saccharide, or chemically degradable by having an ester or disulfide linkage.

In other embodiments, when the reinforcing component does not possess groups capable of forming a covalent bond with the coacervate, the reinforcing component enhance the mechanical properties of the coacervate by occupying or filling gaps in the coacervate. In this embodiment, the reinforcing component is physically entrapped within the coacervate. Upon removal of solvent such as, for example, water, the reinforcing component forms a rigid internal skeleton, which enhances the mechanical properties of the coacervate.

The complex coacervates described herein can be delivered underwater without dispersing into the water. In various embodiments, when applied to a wet substrate, the complex coacervates spread over the interface rather than beading up. In some embodiments, the complex coacervates bond two adherends together, particularly when the adherends are wet or will be exposed to an aqueous environment. The formation of the interpenetrating network can enhance the mechanical properties of the coacervate including, but not limited to, cohesion (*i.e.,* internal strength), fracture toughness, extensibility, fatigue resistance, elastic modulus, etc. In other words, upon formation of the interpenetrating network, the strength of the bond between the two adherends formed by the coacervate can be increased significantly. The degree of crosslinking that occurs during the curing step can vary depending upon the selection of starting materials.

The polycations and polyanions described herein can be stored as dry powders for extended periods of time. Thus, another aspects of the present teachings also provides kits for making the complex coacervates and adhesives described herein. In some embodiments, the kit comprises (1) a dry polycation and (2) a dry polyanion. In certain embodiments, the kit further comprises (3) a reinforcing component and, optionally, (4) an initiator and optional coinitiator. In other embodiments, the kit comprises (1) a dry mixture of polycation and a polyanion, (2) a reinforcing component, and (3) an initiator and optional coinitiator. In a further embodiment, the kit comprises (1) a dry polycation, (2) a dry polyanion, and (3) a reinforcing component, and wherein an initiator and optional coinitiator are covalently attached to the polycation and/or polyanion.

The kits can include additional components as needed such as, for example, an oxidant as described herein. When stored as dried powders, water with or without reinforcing component can be added to the polycation and/or polyanion to produce the coacervate. In some embodiments, prior to lyophilizing the polycation and polyanion in order to produce a dry powder, the pH of the polycation and polyanion is adjusted such that when they are admixed in water the desired pH is produced without the addition of acid or base. For example, excess base can be present in the polycation powder which upon addition of water adjusts the pH accordingly.

The complex coacervates described herein can be applied to a number of different biological substrates. The substrate can be contacted *in vitro* or *in vivo.* The rate of curing can be modified accordingly based upon the selection and amount of initiator used. In the case when the polyanion and polycation are capable of crosslinking with one another, the rate of crosslinking can be controlled by for example pH and the presence of an oxidant or other agents that facilitate crosslinking.

One approach for applying the complex coacervate to the substrate involves the use of a multi-compartment syringe. In some embodiments, a double-compartment or -barrel syringe can be used. For example, one component can hold a mixture of the polycation and polyanion as a dry powder and the second compartment hold a solution of the polymerizable monomer. Either or both compartments can hold additional components such as the polymerization initiator, fillers, and the like. Upon mixing of the dry polycation and polyanion with the solution of polymerizable monomer, the complex coacervate is produced on site. Thus, in this embodiment, the complex coacervate is applied at distinct and specific regions of the substrate.

The coacervates can have low initial viscosity, specific gravity greater than one, low interfacial tension in an aqueous environment, contain a significant fraction of water by weight. Without wishing to be bound a specific theory, all of the above properties are believed to contribute to their ability to adhere to a wet surface. The properties of the complex coacervates described herein make them ideal for wet or underwater applications such as the administration to a subject.

In addition, in some embodiments, the coacervates are water-borne, thus eliminating the need for potentially toxic solvents. In some embodiments, despite being water-borne, they are phase separated from water. In various embodiments, the complex coacervate is underwater without dispersing. The complex coacervates are dimensional stable after crosslinking so that when applied in a wet physiological environment they do not swell. Without wishing to be bound by any particular theory, it believed that the lack of swelling, *i.e.,* absorption of water, is due to the phase-separated nature of the copolymer network. Dimensional stability can be an advantage over tissue adhesives/sealants based on crosslinked PEG hydrogels. The bonding (*i.e.,* crosslinking) of the complex coacervates can generate low heat production during setting, which can prevent damage to living tissue.

The complex coacervates of the invention have antimicrobial activity and, thus, can be used to prevent or treat infection caused by bacteria, fungi, yeast, or protozoan. In some aspects of the present disclosure, the complex coacervates are applied to an infection of a subject in need thereof. For example, the complex coacervates can be part of wound dressing. In certain aspects of the present disclosure, the infection occurs in epidermis, dermis, or hypodermis. Accordingly, the complex coacervates can be used topically. In certain aspects of the present disclosure, the infection occurs in muscle and related tissues (*e.g.,* muscle, ligaments, tendons). In certain aspects of the present disclosure, the infection occurs in certain body cavity. For example, the infection occurs in the thoracic cavity, abdominal cavity, pelvic cavity, cranial cavity, or spinal cavity. In certain aspects of the present disclosure, the infection occurs in certain internal organs. For example, the infection occurs in stomach, intestines, bronchi, lung, bladder, blood vessels, heart, ovaries, fallopian tubes, uterus, vagina, and cartilage. Accordingly, the complex coacervates can be used internally.

The complex coacervates and adhesives produced there from can be used in a variety of surgical procedures. For example, the complex coacervates described herein and adhesives produced therefrom can be used to treat ocular wounds caused by trauma or by the surgical procedures. In some aspects of the present disclosure, the complex coacervates and adhesives produced therefrom are used to repair a corneal or schleral laceration in a subject. In other aspects of the present disclosure, complex coacervates are used to facilitate healing of ocular tissue damaged from a surgical procedure (*e.g.,* glaucoma surgery or a corneal transplant). The methods disclosed in U.S. Published Application No. 2007/0196454 can be used to apply the coacervates described herein to different regions of the eye.

In other aspects of the present disclosure, the complex coacervates and adhesives produced therefrom can be used to inhibit blood flow in a blood vessel of a subject. In general, the complex coacervate is injected into the vessel followed by polymerizing the polymerizable monomer as described above to partially or completely block the vessel. This method has numerous applications including hemastasis or the creation of an artificial embolism to inhibit blood flow to a tumor or aneurysm or other vascular defect.

The complex coacervates described herein can be used to seal the junction between skin and an inserted medical device such as catheters, electrode leads, needles, cannulae, osseo-integrated prosthetics, and the like. In this aspect of the present disclosure, the coacervates prevent infection at the entry site when the device is inserted in the subject. In other aspects of the present disclosure, the coacervates are applied to the entry site of the skin after the device has been removed in order to expedite wound healing and prevent further infection.

In other aspects of the present disclosure, the complex coacervates described herein are used to close or seal a puncture in an internal tissue or membrane. In certain medical and surgical applications, internal tissues or membranes are punctured or incised, and may need to be subsequently sealed in order to avoid additional complications. In various aspects of the present disclosure, the puncture or incision is in an internal organ. For example, the puncture or incision is in a blood vessel, an intestine, stomach, a kidney, bladder, uterus, lung, or diaphragm. Thus, the complex coacervates can be applied to the puncture or incision to seal the puncture and expedite the healing and prevent further infection.

In various aspects of the present disclosure, the complex coacervates described herein are used as anastomosis. For example, the complex coacervates can be used to connect/reconnect two or more blood vessels, two or more segments in the gastrointestinal tract, two or more segments in the urinary tract, two nerve tissues, two segments in the fallopian tube, or two segments in the vas deferens. In some aspects of the present disclosure, the complex coacervates are used as anastomosis and to expedite the healing and prevent further infection.

In various aspects of the present disclosure, the complex coacervates and adhesives produced therefrom are used to repair a number of different bone fractures and breaks. Without wishing to be bound by any particular theory, it is believed that the coacervates adhere to bone (and other minerals) through several mechanisms. The surface of the bone's hydroxyapatite mineral phase (Ca₅(PO₄)₃(OH)) is an array of both positive and negative charges. The negative groups present on the polyanion (*e.g.,* phosphate groups) can interact directly with the positive surface charges or it can be bridged to the negative surface charges through the cationic groups on the polycation and/or multivalent cations. Likewise, direct interaction of the polycation with the negative surface charges would contribute to adhesion. Additionally, when the polycation and/or polyanion contain catechol moieties, they can facilitate the adhesion of the coacervate to readily wet hydroxyapatite. Other adhesion mechanisms include direct bonding of unoxidized crosslinkeable group (*e.g.,* ortho-dihydroxyphenyl compounds or other catechols) to hydroxyapatite. Alternatively, oxidized crosslinkable group can couple to nucleophilic sidechains of bone matrix proteins.

Examples of such breaks include a complete fracture, an incomplete fracture, a linear fracture, a transverse fracture, an oblique fracture, a compression fracture, a spiral fracture, a comminuted fracture, a compacted fracture, or an open fracture. In some aspects of the present disclosure, the fracture is an intra-articular fracture or a craniofacial bone fracture. Fractures such as intra-articular fractures are bony injuries that extend into and fragment the cartilage surface. The complex coacervates and adhesives may aid in the maintenance of the reduction of such fractures, allow less invasive surgery, reduce operating room time, reduce costs, and provide a better outcome by reducing the risk of post-traumatic arthritis.

In other aspects of the present disclosure, the complex coacervates and adhesives produced therefrom are used to join small fragments of highly comminuted fractures. In this aspect of the present disclosure, small pieces of fractured bone is adhered to an existing bone. In some aspects of the present disclosure, the complex coacervate is injected in small volumes to create spot welds as described above in order to fix the fracture rather than filling the entire crack followed by curing the complex coacervate. The small biocompatible spot welds would minimize interference with healing of the surrounding tissue and would not necessarily have to be biodegradable. In this respect it would be similar to permanently implanted hardware.

In other aspects of the present disclosure, the complex coacervates described herein and adhesives produced therefrom are used to secure a patch to bone and other tissues such as, for example, cartilage, ligaments, tendons, soft tissues, organs, and synthetic derivatives of these materials. In some aspects of the present disclosure, the patch is a tissue scaffold or other synthetic materials or substrates typically used in wound healing applications. Using the complexes and spot welding techniques described herein, the complex coacervates and adhesives produced therefrom can be used to position biological scaffolds in a subject. Small adhesive tacks composed of the complex coacervates described herein would not interfere with migration of cells or transport of small molecules into or out of the scaffold. In certain aspects of the present disclosure, the scaffold contains one or more drugs that facilitate growth or repair of the bone and tissue. In other aspects of the present disclosure, the scaffold includes additional drugs that prevent infection such as, for example, antibiotics. For example, the scaffold can be coated with the drug or, in the alternative, the drug can be incorporated within the scaffold so that the drug elutes from the scaffold over time.

In various aspects of the present disclosure, the complex coacervates described herein and adhesives produced therefrom have numerous dental applications. For example, the complex coacervates can be used to seal breaks or cracks in teeth, for securing crowns, or allografts, or seating implants and dentures. The complex coacervate can be applied to a specific points in the mouth (e.g., jaw, sections of a tooth) followed by attaching the implant to the substrate and subsequent curing.

In other aspects of the present disclosure, the complex coacervates and adhesives produced therefrom adhere a substrate to a tissue. The complex coacervate can be applied to the metal substrate, the tissue, or both prior to adhering the substrate to the tissue. In certain aspects of the present disclosure, the crosslinkable groups present on the polycation or polyanion form a strong bond with the implant. In particular aspects of the present disclosure, the complex coacervates are used to bond a substrate to bone. For example, the substrate can be made of titanium oxide, stainless steel, or other metals commonly used to repair fractured bones. In other aspects of the present disclosure, the substrate is a fabric (e.g., an internal bandage), a tissue graft, or a wound healing material.

It is also contemplated that the complex coacervates and produced therefrom can encapsulate one or more bioactive agents. The bioactive agents can be any drug including, but not limited to, antibiotics, pain relievers, immune modulators, growth factors, enzyme inhibitors, hormones, mediators, messenger molecules, cell signaling molecules, receptor agonists, or receptor antagonists.

In other aspects of the present disclosure, the bioactive agent is a nucleic acid. The nucleic acid can be an oligonucleotide, deoxyribonucleic acid (DNA), ribonucleic acid (RNA), or peptide nucleic acid (PNA). The nucleic acid of interest can be nucleic acid from any source, such as a nucleic acid obtained from cells in which it occurs in nature, recombinantly produced nucleic acid, or chemically synthesized nucleic acid. For example, the nucleic acid can be cDNA or genomic DNA or DNA synthesized to have the nucleotide sequence corresponding lo that of naturally-occurring DNA. The nucleic acid can also be a mutated or altered form of nucleic acid (*e.g.,* DNA that differs from a naturally occurring DNA by an alteration, deletion, substitution or addition of at least one nucleic acid residue) or nucleic acid that does not occur in nature.

In other aspects of the present disclosure, the bioactive agent is used in tissue treatment applications. For example, the bioactive agent can be used to promote tissue growth/regrowth, cell attachment, cell migration, cell recapitulation, etc. In certain aspects of the present disclosure, the bioactive agent is used in bone tissue treatment applications. For example, the bioactive agent can be bone morphogenetic proteins (BMPs) and prostaglandins. When the bioactive agent is used to treat osteoporosis, bioactive agents known in the art such as, for example, bisphonates, can be delivered locally to the subject by the complex coacervates and adhesives described herein.

In certain aspects of the present disclosure, the fillers used to produce the coacervate also possess bioactive properties. For example, when the filler is a silver particle, the particle can also behave as an anti-bacterial agent. The rate of release can be controlled by the selection of the materials used to prepare the complex as well as the charge of the bioactive agent if the agent is a salt. Thus, in this aspect of the present disclosure, the insoluble solid can perform as a localized controlled drug release depot. It may be possible to simultaneously fix tissue, including bones, as well as deliver bioactive agents to provide greater patient comfort and/or prevent infections.

Alternatively, the complex coacervates described herein can be used to adhere a scaffold or patch to the tissue or membrane.

In some embodiments, the complex coacervates are used in tissue engineering *in vitro.* In some aspects of the present disclosure, the complex coacervates are used in tissue engineering *in vivo.* For example, the complex coacervates can be used to make a structure, for example, by a known process. In certain aspects of the present disclosure, the known process is selected from injection molding, extrusion, compressing molding, transfer molding, laminating, vacuum forming, and rotational molding. In certain aspects of the present disclosure, the known process is a rapid prototyping process. The rapid prototyping process can be selected from stereolithography, laminated object manufacturing, and 3-D printing. The structure obtained from the above process can be further modified for its application. In various aspects of the present disclosure, the structure is a scaffold. In some aspects of the present disclosure, the structure allows or facilitates cell attachment and migration. In certain aspects of the present disclosure, the structure is implantable. The structure is biodegradable.

The following examples are put forth to provide those of ordinary skill in the art with a complete disclosure and description of how the compounds, compositions, and methods described and claimed herein are made and evaluated, and are intended to be purely exemplary and are not intended to limit the scope of what the inventors regard as their invention. Efforts have been made to ensure accuracy with respect to numbers (*e.g.,* amounts, temperature, etc.) but some errors and deviations should be accounted for. Unless indicated otherwise, parts are parts by weight, temperature is in °C or is at ambient temperature, and pressure is at or near atmospheric. There are numerous variations and combinations of reaction conditions, *e.g.,* component concentrations, desired solvents, solvent mixtures, temperatures, pressures and other reaction ranges and conditions that can be used to optimize the product purity and yield obtained from the described process. Only reasonable and routine experimentation will be required to optimize such process conditions.

### Coacervate Formation

The adhesive coacervate was formed with two oppositely charged polymer systems. First, a positively charged chitosan derivative (CA) and a negatively charged polyphosphate-co-methacrylic acid (PPMA) containing 20 mol% dopamide side groups (polyphosphodopa) were mixed at a selected pH to form a complex coacervate.

A 50 mg/ml aqueous polycation solution was prepared by dissolving a selected CA derivative in deionized (D.I.) water. A 50 mg/ml aqueous polyanion solution was prepared by dissolving a selected PPMA in D.I. water. Magnesium chloride was added to the polyanion solution to obtain a Mg⁺² to phosphate molar ratio of 0.8. The pH of the CA solution and polyanion solution was adjusted to the desired pH by addition of NaOH. While stirring, the CA solution was added dropwise into the PPMA solution to form the complex coacervate. The solution appeared cloudy at first. Within a few minutes the coacervate phase settled to the bottom with a clear supernatant at the top. The complex coacervate was collected by removing the supernatant from the top.

### Rheological Properties

The effect of CA composition on complex coacervate viscosity at a pH of 7.4 was examined. Four different CA derivatives were studied: two derivatives with low molecular weight (one having a high arginine content, and the other having a low arginine content) and two derivatives with high molecular weights (one having a high arginine content and the other having a low arginine content). Table 1 summarizes the properties of the resulting complex coacervates.

**Table 1**

| CA | MW (kD) | Arginine (%) | Amine (%) | Total Charge (%) | Complex Viscosity (Pa.s) | G' (Pa) | G" (Pa) | Phase |
|---|---|---|---|---|---|---|---|---|
| 081106SJRBC06 | 65.1 | 11 | 77 | 19.61 | 75.6 | 285 | 380 | viscous fluid |
| 081110SJRBC07 | 33.5 | 10 | 78 | 18.72 | 6.43 | 15.6 | 37.3 | viscous fluid |
| 100928HI04 | 27.6 | 22 | 68 | 29.6 | 24.9 | 45.3 | 150 | viscous fluid |
| 101116HI08 | 71 | 23 | 67 | 30.94 | 1360 | 6840 | 5120 | solid |

The effect of pH on coacervate formation and rheological properties was examined using complex coacervates formulated with CA of low molecular weight and low arginine content of 11 % and amine content of 78 %. Table 2 summarizes the properties of the complex coacervates formed under different pH.

**Table 2**

| | | | | | | | |
|---|---|---|---|---|---|---|---|
| Chitosan-Arginine | pH | Total Charge (%) | CC Phase | G' (Pa) | G" (Pa) | Complex Viscosity (Pa.s) | CC Density (mg/mL) |
| | 5.4 | 82 | high viscous fluid | 2800 | 3340 | 697 | 324 |
| | 6.4 | 49 | high viscous fluid | 1680 | 2450 | 476 | 297 |
| | 7.4 | 19 | viscous fluid | 25.5 | 83 | 14 | 202 |
| | 8.4 | 11 | viscous fluid | 16.4 | 40.5 | 6.8 | 104 |
| | 9.5 | 5 | clear solution | | | | |

### Adhesive Curing

Complex coacervates using CA of low molecular weight and low arginine content were cured by adding a cross linking agent. EDC (1-ethyl-3-(3-dimethylaminopropyl)carbodiimide) was added to the complex coacervate to obtain an EDC to COOH ratio of 2. The curing kinetics were monitored by complex rheology. Figure 4 shows the storage and loss modulus of the curing adhesive. The crossing point of the two curves represents the gelling point and occurs at roughly 1-minute for this crosslinking system.

### Cured Adhesive Bond Strength

To test the adhesive strength of the cured complex coacervate using a lap-shear test, 5052 aluminum substrates of dimensions 0.5 x 5 cm were used. The substrates were polished with 600 grit sand paper followed by cleaning with methanol under sonication for 10 minutes twice, air-dried, dipped into sulfuric acid for 15 minutes, rinsed with deionized water, and stored in deionized water until used for bonding tests.

Curing agent was added to the coacervate before applying to a 0.5 x 0.5 cm area of one end of an aluminum substrate. Another aluminum substrate was contacted with the first substrate and held in place at a fixed spacing using a custom jig. All bonding and curing was conducted with D.I. water maintained at a controlled temperature of 37 °C. The substrates are allowed to cure for at least 6 hours, and are tested with an Instron testing system with a 100 N load cell and a cross-head speed of 0.5 cm/min. After failing, the cured adhesive complex coacervate was measured to have a bond strength of about 600 kPa.

## Claims

1. A composition comprising a polycation and a polyanion, wherein the composition has antimicrobial activity and wherein the polycation comprises a saccharide of formula (I): 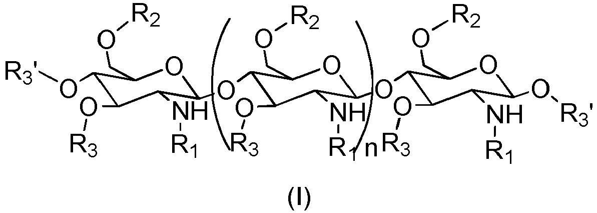 wherein:
n is an integer ranging from 20 and 6000,
R₁ at each occurrence independently is selected from hydrogen, alkyl, and -C(O)-R₄, wherein at least one R₁ is selected from 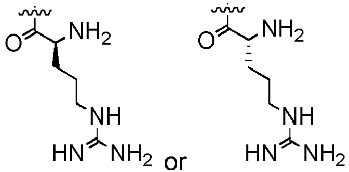
R₂ at each occurrence independently is selected from hydrogen, alkyl, and -C(O)-R₄;
R₃ at each occurrence independently is selected from hydrogen, alkyl, and -C(O)-R₄; and
R₃' at each occurrence independently is selected from hydrogen, alkyl, and -C(O)-R₄;
wherein R₄ at each occurrence independently is selected from hydrogen, alkyl, alkenyl, alkynyl alkoxy, cycloalkyl, aryl, heterocyclyl, and heteroaryl;
or a salt thereof,
wherein the polyanion comprises a polyacrylate having one or more pendant phosphate groups;
wherein the polycation, the polyanion, or each of the polyanion and polycation comprises a crosslinkable group; and
wherein the composition is biodegradable.

2. The composition of claim 1, wherein the polycation has antimicrobial activity.

3. The composition of claim 1 or 2, wherein the polycation comprises one or more amino groups.

4. The composition of any one of claims 1 to 3, wherein the polycation comprises a fragment of formula (III): 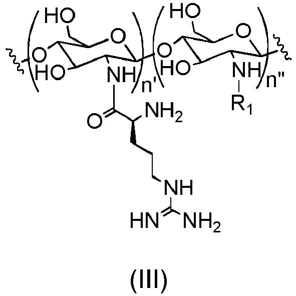 wherein:
n' and n" each independently is an integer ranging from 0 and 6000, wherein 1 ≤ (n' + n") ≤ 6000, and
R₁ at each occurrence independently is hydrogen or -C(O)-R₄ and R₄ is alkyl.

5. The composition of any one of claims 1 to 4, wherein the polyanion further comprises a sulfate, a sulfonate, a borate, a boronate, or a phosphonate group.

6. The composition of any one of claims 1 to 4, wherein the polyanion comprises a fragment of formula (VII): 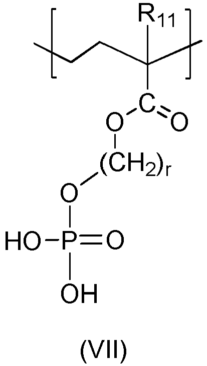 wherein:
R₁₁ at each occurrence independently is selected from hydrogen or alkyl; and
r at each occurrence independently is an integer from 1 to 10;
or a pharmaceutically acceptable salt thereof.

7. The composition of claim 6, wherein at least one R₁₁ is methyl and at least one r is 2.

8. The composition of any one of claims 1 to 7, further comprising:
a multivalent cation; and/or,
a reinforcing component; and/or,
an initiator and optionally a co-initiator; and/or,
a bioactive agent.

9. The composition of any one of claims 1 to 8, wherein the composition is a complex coacervate and/or an adhesive complex coacervate.

10. The composition of any one of claims 1 to 9 for use in therapy.

11. The composition of any one of claims 1 to 9 for use in a method of treating or preventing an infection, the method comprising applying the composition of any one of claims 1 to 9 to an infection on a subject in need thereof; optionally, wherein the infection is selected from a bacterial infection and a fungal infection.

12. The composition of any one of claims 1 to 9 for use in a method of attaching an implant to a tissue, the method comprising applying to an implant or a tissue of a subject in need thereof the composition of any one of claims 1 to 9, and contacting the implant with the tissue; optionally, wherein the tissue is a bone tissue, a muscle tissue, nerve tissue or an epithelial tissue.

13. The composition of any one of claims 1 to 9 for use in a method of treating a defect, the method comprising applying the composition to a defect in a subject in need thereof; optionally, wherein the defect is selected from a bone defect, a surgical incision, a puncture, a gap between two tissue planes, and a gap between two tissues; further optionally, wherein the defect is a bone fracture, a surgical incision, a puncture in an internal organ, optionally wherein the internal organ is a blood vessel, an intestine, stomach, a kidney, bladder, uterus, lung or diaphragm, a corneal laceration or a sclera laceration.

14. A method of making the composition of any one of claims 1 to 9 comprising the steps of (1) mixing a polycation solution and a polyanion solution, wherein the polycation has antimicrobial activity; and (2) adjusting the pH of the polycation solution or the polyanion solution to a desired value.

15. A kit for forming a composition according to any one of claims 1 to 9, comprising a polycation and a polyanion, wherein the polycation has antimicrobial activity and wherein the polycation comprises a saccharide of formula (I): 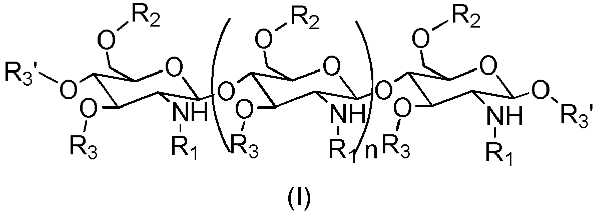 wherein:
n is an integer ranging from 20 and 6000,
R₁ at each occurrence independently is selected from hydrogen, alkyl, and -C(O)-R₄, wherein at least one R₁ is selected from 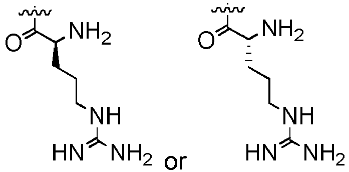
R₂ at each occurrence independently is selected from hydrogen, alkyl, and -C(O)-R₄;
R₃ at each occurrence independently is selected from hydrogen, alkyl, and -C(O)-R₄; and
R₃' at each occurrence independently is selected from hydrogen, alkyl, and -C(O)-R₄;
wherein R₄ at each occurrence independently is selected from hydrogen, alkyl, alkenyl, alkynyl alkoxy, cycloalkyl, aryl, heterocyclyl, and heteroaryl;
or a salt thereof,
wherein the polyanion comprises a polyacrylate having one or more pendant phosphate groups;
wherein the polycation, the polyanion, or each of the polyanion and polycation comprises a crosslinkable group.

16. A method of making a structure comprising providing the composition of any one of claims 1 to 9 and using a process to make a structure.

17. A structure obtainable by the method of claim 16.

## Patentansprüche

1. Zusammensetzung, die ein Polykation und ein Polyanion umfasst, wobei die Zusammensetzung antimikrobielle Aktivität aufweist und wobei das Polykation ein Saccharid von Formel (I) umfasst: 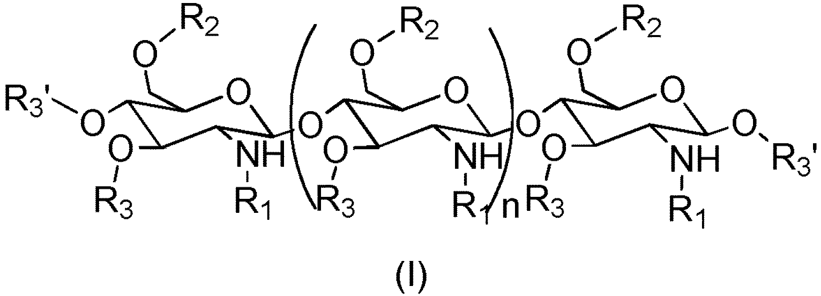 wobei:
N eine ganze Zahl im Bereich von 20 bis 6000 ist,
R₁ bei jedem Vorkommen unabhängig ausgewählt ist aus Wasserstoff, Alkyl und - C(O)-R₄, wobei wenigstens ein R₁ ausgewählt ist aus 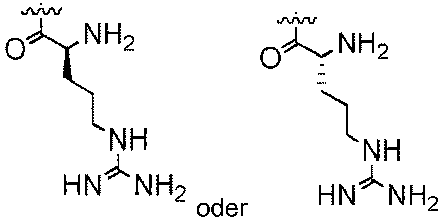
R₂ bei jedem Vorkommen unabhängig ausgewählt ist aus Wasserstoff, Alkyl und - C(O)-R4;
R₃ bei jedem Vorkommen unabhängig ausgewählt ist aus Wasserstoff, Alkyl und - C(O)-R4; und
R₃' bei jedem Vorkommen unabhängig ausgewählt ist aus Wasserstoff, Alkyl und - C(O)-R₄;
wobei R₄ bei jedem Vorkommen unabhängig ausgewählt ist aus Wasserstoff, Alkyl, Alkenyl, Alkynyl, Alkoxy, Cycloalkyl, Aryl, Heterocyclyl und Heteroaryl;
oder ein Salz davon,
wobei das Polyanion ein Polyacrylat mit einer oder mehreren anhängigen Phosphatgruppen umfasst;
wobei das Polykation, das Polyanion oder das Polyanion und das Polykation jeweils eine vernetzbare Gruppe umfasst/umfassen; und
wobei die Zusammensetzung biologisch abbaubar ist.

2. Zusammensetzung nach Anspruch 1, wobei das Polykation antimikrobielle Aktivität aufweist.

3. Zusammensetzung nach Anspruch 1 oder 2, wobei das Polykation ein oder mehrere Aminogruppen umfasst.

4. Zusammensetzung nach einem der Ansprüche 1 bis 3, wobei das Polykation ein Fragment von Formel (III) umfasst: 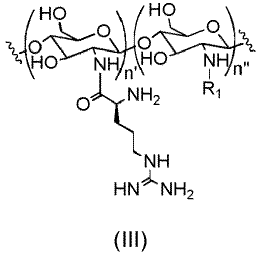 wobei:
n' und n" jeweils unabhängig eine ganze Zahl im Bereich von 0 bis 6000 sind, wobei 1 ≤ (n' + n") ≤ 6000 ist, und
R₁ bei jedem Vorkommen unabhängig Wasserstoff oder -C(O)-R₄ ist und R₄ Alkyl ist.

5. Zusammensetzung nach einem der Ansprüche 1 bis 4, wobei das Polyanion ferner ein Sulfat, ein Sulfonat, ein Borat, ein Boronat oder eine Phosphonatgruppe umfasst.

6. Zusammensetzung nach einem der Ansprüche 1 bis 4, wobei das Polyanion ein Fragment von Formel (VII) umfasst: 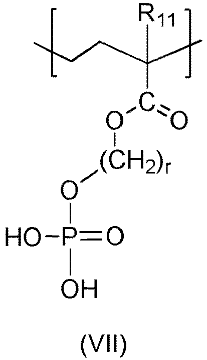 wobei:
R₁₁ bei jedem Vorkommen unabhängig ausgewählt ist aus Wasserstoff oder Alkyl; und
r bei jedem Vorkommen unabhängig eine ganze Zahl von 1 bis 10 ist;
oder ein pharmazeutisch akzeptables Salz davon.

7. Zusammensetzung nach Anspruch 6, wobei wenigstens ein R₁₁ Methyl ist und wenigstens ein r 2 ist.

8. Zusammensetzung nach einem der Ansprüche 1 bis 7, die ferner Folgendes umfasst:
ein multivalentes Kation; und/oder
eine Verstärkungskomponente; und/oder
einen Initiator und optional einen Co-Initiator; und/oder
ein bioaktives Mittel.

9. Zusammensetzung nach einem der Ansprüche 1 bis 8, wobei die Zusammensetzung ein komplexes Koazervat und/oder ein adhäsives komplexes Koazervat ist.

10. Zusammensetzung nach einem der Ansprüche 1 bis 9 zur Verwendung in der Therapie.

11. Zusammensetzung nach einem der Ansprüche 1 bis 9 zur Verwendung in einem Verfahren zur Behandlung oder Verhütung einer Infektion, wobei das Verfahren das Anwenden der Zusammensetzung nach einem der Ansprüche 1 bis 9 bei einer Infektion an einem dies benötigenden Subjekt beinhaltet; wobei die Infektion optional aus einer bakteriellen Infektion und einer Pilzinfektion ausgewählt ist.

12. Zusammensetzung nach einem der Ansprüche 1 bis 9 zur Verwendung bei einem Verfahren zum Befestigen eines Implantats an einem Gewebe, wobei das Verfahren das Anwenden der Zusammensetzung nach einem der Ansprüche 1 bis 9 an einem Implantat oder einem Gewebe eines dies benötigenden Subjekts und das Inkontaktbringen des Implantats mit dem Gewebe beinhaltet; wobei das Gewebe optional ein Knochengewebe, ein Muskelgewebe, ein Nervengewebe oder ein Epithelgewebe ist.

13. Zusammensetzung nach einem der Ansprüche 1 bis 9 zur Verwendung bei einem Verfahren zur Behandlung eines Defekts, wobei das Verfahren das Anwenden der Zusammensetzung an einem Defekt bei einem dies benötigenden Subjekt beinhaltet; wobei der Defekt optional ausgewählt ist aus einem Knochendefekt, einem chirurgischen Einschnitt, einer Punktion, einem Spalt zwischen zwei Gewebeebenen und einem Spalt zwischen zwei Geweben; wobei der Defekt ferner optional Folgendes ist: eine Knochenfraktur, ein chirurgischer Einschnitt, eine Punktion in einem inneren Organ, wobei das innere Organ optional ein Blutgefäß, ein Darm, ein Magen, eine Niere, Blase, Gebärmutter, Lunge oder ein Zwerchfell ist, eine Hornhautverletzung oder eine Lederhautverletzung.

14. Verfahren zur Herstellung der Zusammensetzung nach einem der Ansprüche 1 bis 9, das die folgenden Schritte beinhaltet: (1) Mischen einer Polykationlösung und einer Polyanionlösung, wobei das Polykation antimikrobielle Aktivität aufweist; und (2) Einstellen des pH-Werts der Polykationlösung oder der Polyanionlösung auf einen gewünschten Wert.

15. Kit zum Bilden einer Zusammensetzung nach einem der Ansprüche 1 bis 9, der ein Polykation und ein Polyanion umfasst, wobei das Polykation antimikrobielle Aktivität aufweist und wobei das Polykation ein Saccharid von Formel (I) umfasst: 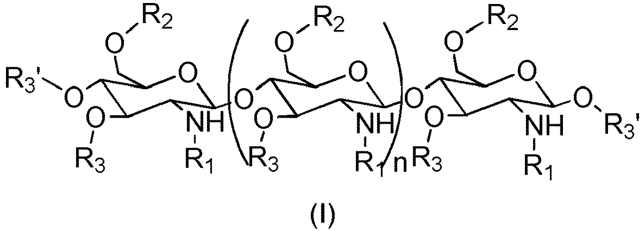 wobei:
n eine ganze Zahl im Bereich von 20 bis 6000 ist,
R₁ bei jedem Vorkommen unabhängig ausgewählt ist aus Wasserstoff, Alkyl und - C(O)-R4, wobei wenigstens ein R₁ ausgewählt ist aus 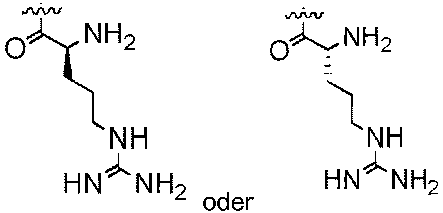
R₂ bei jedem Vorkommen unabhängig ausgewählt ist aus Wasserstoff, Alkyl und -C(O)-R₄;
R₃ bei jedem Vorkommen unabhängig ausgewählt ist aus Wasserstoff, Alkyl und -C(O)-R₄; und
R₃' bei jedem Vorkommen unabhängig ausgewählt ist aus Wasserstoff, Alkyl und -C(O)-R₄;
wobei R₄ bei jedem Vorkommen unabhängig ausgewählt ist aus Wasserstoff, Alkyl, Alkenyl, Alkynyl, Alkoxy, Cycloalkyl, Aryl, Heterocyclyl und Heteroaryl; oder ein Salz davon,
wobei das Polyanion ein Polyacrylat mit einer oder mehreren anhängigen Phosphatgruppen umfasst;
wobei das Polykation, das Polyanion oder das Polyanion und das Polykation jeweils eine vernetzbare Gruppe umfasst/umfassen.

16. Verfahren zur Herstellung einer Struktur, das das Bereitstellen der Zusammensetzung nach einem der Ansprüche 1 bis 9 und das Anwenden eines Verfahrens zur Herstellung einer Struktur beinhaltet.

17. Struktur, die mit dem Verfahren nach Anspruch 16 erhältlich ist.

## Revendications

1. Une composition comprenant un polycation et un polyanion, la composition ayant une activité antimicrobienne et le polycation comprenant un saccharide de formule (I) : 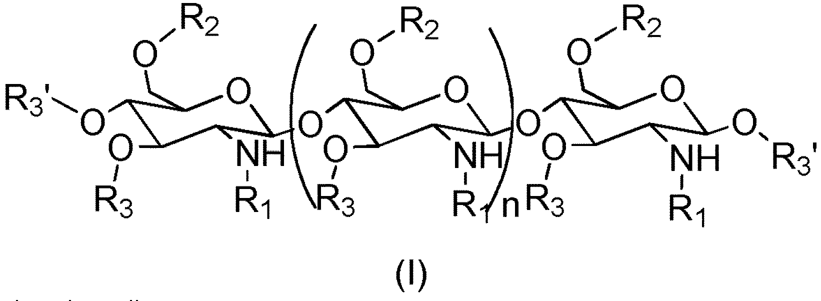 dans laquelle :
N est un entier allant de 20 à 6000,
R₁ à chaque occurrence indépendamment est choisi parmi l'hydrogène, un alkyle et le -C(O)-R₄,
dans laquelle au moins un R₁ est choisi entre 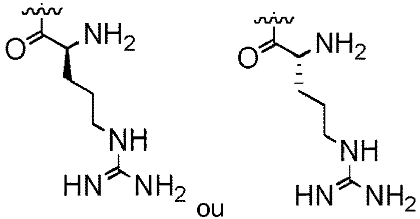
R₂ à chaque occurrence indépendamment est choisi parmi l'hydrogène, un alkyle et le -C(O)-R₄ ;
R₃ à chaque occurrence indépendamment est choisi parmi l'hydrogène, un alkyle et le -C(O)-R₄ ; et
R₃' à chaque occurrence indépendamment est choisi parmi l'hydrogène, un alkyle et le -C(O)-R₄ ;
dans laquelle R₄ à chaque occurrence indépendamment est choisi parmi l'hydrogène, un alkyle, un alkényle, un alkynyle, un alkoxy, un cycloalkyle, un aryle, un hétérocyclyle et un hétéroaryle ;
ou un sel de ceux-ci ;
dans laquelle le polyanion comprend un polyacrylate présentant un ou plusieurs groupes phosphate pendants ;
dans laquelle le polycation, le polyanion ou chacun du polyanion et du polycation comprend un groupe réticulable ; et
dans laquelle la composition est biodégradable.

2. La composition de la revendication 1, dans laquelle le polycation a une activité antimicrobienne.

3. La composition de la revendication 1 ou 2, dans laquelle le polycation comprend un ou plusieurs groupes amino.

4. La composition de n'importe laquelle des revendications 1 à 3, dans laquelle le polycation comprend un fragment de formule (III) : 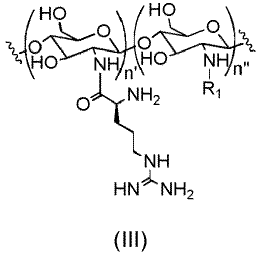 dans laquelle :
n' et n" chacun indépendamment est un entier allant de 0 à 6000, dans laquelle 1 ≤ (n' + n") ≤ 6000, et
R₁ à chaque occurrence indépendamment est l'hydrogène ou le -C(O)-R₄ et R₄ est un alkyle.

5. La composition de n'importe laquelle des revendications 1 à 4, dans laquelle le polyanion comprend en sus un groupe sulfate, sulfonate, borate, boronate ou phosphonate.

6. La composition de n'importe laquelle des revendications 1 à 4, dans laquelle le polyanion comprend un fragment de formule (VII) : 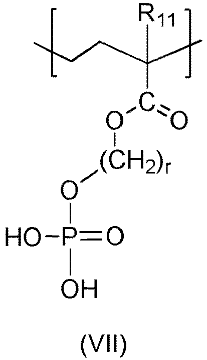 dans laquelle :
R₁₁ à chaque occurrence indépendamment est choisi parmi l'hydrogène ou un alkyle ; et
r à chaque occurrence indépendamment est un entier de 1 à 10 ;
ou un sel pharmaceutiquement acceptable de celui-ci.

7. La composition de la revendication 6, dans laquelle au moins un R₁₁ est le méthyle et au moins un r est 2.

8. La composition de n'importe laquelle des revendications 1 à 7, comprenant en sus :
un cation multivalent ; et/ou
un composant de renfort ; et/ou
un initiateur et éventuellement un co-initiateur ; et/ou
un agent bioactif.

9. La composition de n'importe laquelle des revendications 1 à 8, dans laquelle la composition est un coacervat complexe et/ou un coacervat complexe adhésif.

10. La composition de n'importe laquelle des revendications 1 à 9 pour utilisation dans un traitement.

11. La composition de n'importe laquelle des revendications 1 à 9 pour utilisation dans un procédé de traitement ou de prévention d'une infection, le procédé comprenant les étapes consistant à appliquer la composition de n'importe laquelle des revendications 1 à 9 à une infection sur un sujet le nécessitant ; éventuellement, dans laquelle l'infection est sélectionnée parmi une infection bactérienne et une infection fongique.

12. La composition de n'importe laquelle des revendications 1 à 9 pour utilisation dans un procédé consistant à attacher un implant à un tissu, le procédé comprenant les étapes consistant à appliquer à un implant ou un tissu d'un sujet le nécessitant la composition de n'importe laquelle des revendications 1 à 9, et mettre en contact l'implant avec le tissu ; éventuellement, dans laquelle le tissu est un tissu osseux, un tissu musculaire, un tissu nerveux ou un tissu épithélial.

13. La composition de n'importe laquelle des revendications 1 à 9 pour utilisation dans un procédé de traitement d'un défaut, le procédé comprenant les étapes consistant à appliquer la composition à un défaut chez un sujet le nécessitant ; éventuellement dans laquelle le défaut est sélectionné parmi un défaut osseux, une incision chirurgicale, une perforation, un écart entre deux plans de tissus et un écart entre deux tissus ; éventuellement, en sus, dans laquelle le défaut est une fracture osseuse, une incision chirurgicale, une perforation dans un organe interne, éventuellement dans laquelle l'organe interne est un vaisseau sanguin, un intestin, l'estomac, un rein, la vessie, l'utérus, un poumon ou le diaphragme, une lacération de la cornée ou une lacération de la sclérotique.

14. Un procédé pour fabriquer la composition de n'importe laquelle des revendications 1 à 9 comprenant les étapes consistant à (1) mélanger une solution de polycation et une solution de polyanion, dans laquelle le polycation a une activité antimicrobienne ; et (2) ajuster le pH de la solution de polycation ou de la solution de polyanion jusqu'à une valeur voulue.

15. Un kit pour former une composition selon n'importe laquelle des revendications 1 à 9, comprenant un polycation et un polyanion, dans laquelle le polycation a une activité antimicrobienne et dans laquelle le polycation comprend un saccharide de formule (I) : 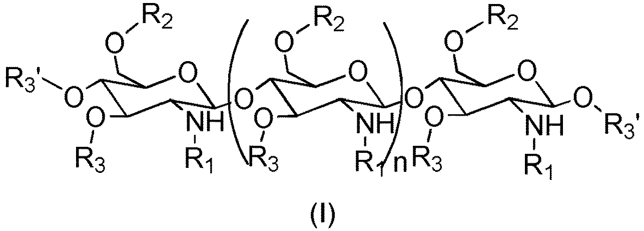 dans laquelle :
n est un entier allant de 20 à 6000,
R₁ à chaque occurrence indépendamment est choisi parmi l'hydrogène, un alkyle et le -C(O)-R₄, dans laquelle au moins un R₁ est sélectionné entre 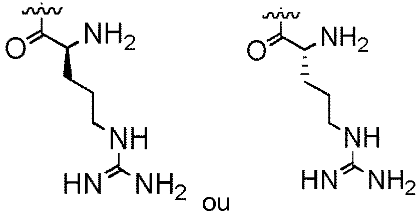
R₂ à chaque occurrence indépendamment est choisi parmi l'hydrogène, un alkyle et le -C(O)-R₄ ;
R₃ à chaque occurrence indépendamment est choisi parmi l'hydrogène, un alkyle et le -C(O)-R₄ ; and
R₃' à chaque occurrence indépendamment est choisi parmi l'hydrogène, un alkyle et le -C(O)-R₄ ;
dans laquelle R₄ à chaque occurrence indépendamment est choisi parmi l'hydrogène, un alkyle, un alkényle, un alkynyle, un alkoxy, un cycloalkyle, un aryle, un hétérocyclyle et un hétéroaryle ; ou un sel de ceux-ci,
dans laquelle the polyanion comprend un polyacrylate présentant un ou plusieurs groupes de phosphate pendants ;
dans laquelle le polycation, le polyanion ou chacun du polyanion et du polycation comprend un groupe réticulable.

16. Un procédé pour fabriquer une structure comprenant l'étape consistant à prévoir la composition de n'importe laquelle des revendications 1 à 9 et utiliser un procédé pour fabriquer une structure.

17. Une structure pouvant être obtenue par le procédé de la revendication 16.


### SEQUENCE LISTING

<110> Kinetic Licensing, Inc.
<120> NEW COMPOSITIONS, THE PREPARATION AND USE THEREOF
<130> UOU.0104PCT
<150> 61/601,804
   <151> 2012-02-22
<160> 26
<170> PatentIn version 3.5
<210> 1
   <211> 6
   <212> PRT
   <213> Artificial Sequence
<220>
   <223> Synthetic peptide
<220>
   <221> MISC-FEATURE
   <222> (3)..(3)
   <223> Xaa is a hydroxyproline
<400> 1
   Gly Phe Xaa Gly Glu Arg
   1               5
<210> 2
   <211> 6
   <212> PRT
   <213> Artificial Sequence
<220>
   <223> Synthetic peptide
<220>
   <221> MISC_FEATURE
   <222> (3)..(3)
   <223> xaa is a hydroxyproline
<400> 2
   Gly Leu Xaa Gly Glu Arg
   1               5
<210> 3
   <211> 6
   <212> PRT
   <213> Artificial Sequence
<220>
   <223> Synthetic peptide
<400> 3
   Gly Ala Ser Gly Glu Arg
   1               5
<210> 4
   <211> 6
   <212> PRT
   <213> Artificial Sequence
<220>
   <223> Synthetic peptide
<400> 4
   Gly Leu Pro Gly Glu Arg
   1               5
<210> 5
   <211> 6
   <212> PRT
   <213> Artificial Sequence
<220>
   <223> Synthetic peptide
<400> 5
   Gly Leu Pro Gly Glu Asn
   1               5
<210> 6
   <211> 6
   <212> PRT
   <213> Artificial Sequence
<220>
   <223> Synthetic peptide
<400> 6
   Gly Leu Pro Gly Glu Lys
   1               5
<210> 7
   <211> 6
   <212> PRT
   <213> Artificial sequence
<220>
   <223> Synthetic peptide
<400> 7
   Gly Arg Pro Gly Glu Arg
   1               5
<210> 8
   <211> 6
   <212> PRT
   <213> Artificial Sequence
<220>
   <223> Synthetic peptide
<400> 8
   Gly Arg Pro Gly Glu Asn
   1               5
<210> 9
   <211> 6
   <212> PRT
   <213> Artificial Sequence
<220>
   <223> Synthetic peptide
<400> 9
   Gly Arg Pro Gly Glu Lys
   1               5
<210> 10
   <211> 6
   <212> PRT
   <213> Artificial Sequence
<220>
   <223> Synthetic peptide
<400> 10
   Gly Phe Pro Gly Glu Arg
   1               5
<210> 11
   <211> 6
   <212> PRT
   <213> Artificial Sequence
<220>
   <223> Synthetic peptide
<400> 11
   Gly Phe Pro Gly Glu Asn
   1               5
<210> 12
   <211> 6
   <212> PRT
   <213> Artificial Sequence
<220>
   <223> Synthetic peptide
<400> 12
   Gly Phe Pro Gly Glu Lys
   1               5
<210> 13
   <211> 12
   <212> PRT
   <213> Artificial Sequence
<220>
   <223> Synthetic peptide
<220>
   <221> MISC-FEATURE
   <222> (2)..(3)
   <223> Xaa is any amino acid
<220>
   <221> MISC-FEATURE
   <222> (5)..(5)
   <223> Xaa is Leu, Arg or Phe
<220>
   <221> MISC_FEATURE
   <222> (6)..(6)
   <223> Xaa is Pro
<220>
   <221> MISC_FEATURE
   <222> (8)..(8)
   <223> xaa is Glu
<220>
   <221> MISC_FEATURE
   <222> (9)..(9)
   <223> xaa is Arg, Lys or Asn
<220>
   <221> MISC-FEATURE
   <222> (11)..(12)
   <223> Xaa is any amino acid
<400> 13
   Gly Xaa Xaa Gly Xaa Xaa Gly Xaa Xaa Gly Xaa Xaa
   1 5 10
<210> 14
   <211> 12
   <212> PRT
   <213> Artificial sequence
<220>
   <223> Synthetic peptide
<220>
   <221> MISC_FEATURE
   <222> (2)..(3)
   <223> Xaa is any amino acid
<220>
   <221> MISC_FEATURE
   <222> (11)..(12)
   <223> Xaa is any amino acid
<400> 14
   Gly Xaa Xaa Gly Leu Pro Gly Glu Arg Gly Xaa Xaa
   1 5 10
<210> 15
   <211> 12
   <212> PRT
   <213> Artificial Sequence
<220>
   <223> Synthetic peptide
<220>
   <221> MISC_FEATURE
   <222> (2)..(3)
   <223> Xaa is any amino acid
<220>
   <221> MISC-FEATURE
   <222> (11)..(12)
   <223> Xaa is any amino acid
<400> 15
   Gly Xaa Xaa Gly Leu Pro Gly Glu Asn Gly Xaa Xaa
   1 5 10
<210> 16
   <211> 12
   <212> PRT
   <213> Artificial Sequence
<220>
   <223> Synthetic peptide
<220>
   <221> MISC_FEATURE
   <222> (2)..(3)
   <223> xaa is any amino acid
<220>
   <221> MISC_FEATURE
   <222> (11)..(12)
   <223> Xaa is any amino acid
<400> 16
   Gly Xaa Xaa Gly Leu Pro Gly Glu Lys Gly Xaa Xaa
   1               5                   10
<210> 17
   <211> 12
   <212> PRT
   <213> Artificial Sequence
<220>
   <223> synthetic peptide
<220>
   <221> MISC_FEATURE
   <222> (2)..(3)
   <223> xaa is any amino acid
<220>
   <221> MISC_FEATURE
   <222> (11)..(12)
   <223> xaa is any amino acid
<400> 17
   Gly Xaa Xaa Gly Arg Pro Gly Glu Arg Gly Xaa Xaa
   1               5                   10
<210> 18
   <211> 12
   <212> PRT
   <213> Artificial sequence
<220>
   <223> Synthetic peptide
<220>
   <221> MISC_FEATURE
   <222> (2)..(3)
   <223> Xaa is any amino acid
<220>
   <221> MISC_FEATURE
   <222> (11)..(12)
   <223> Xaa is any amino acid
<400> 18
   Gly Xaa Xaa Gly Arg Pro Gly Glu Asn Gly Xaa Xaa
   1 5 10
<210> 19
   <211> 12
   <212> PRT
   <213> Artificial Sequence
<220>
   <223> Synthetic peptide
<220>
   <221> MISC_FEATURE
   <222> (2)..(3)
   <223> Xaa is any amino acid
<220>
   <221> MISC_FEATURE
   <222> (11)..(12)
   <223> Xaa is any amino acid
<400> 19
   Gly Xaa Xaa Gly Arg Pro Gly Glu Lys Gly Xaa Xaa
   1 5 10
<210> 20
   <211> 12
   <212> PRT
   <213> Artificial Sequence
<220>
   <223> Synthetic peptide
<220>
   <221> MISC_FEATURE
   <222> (2)..(3)
   <223> xaa is any amino acid
<220>
   <221> MISC_FEATURE
   <222> (11)..(12)
   <223> Xaa is any amino acid
<400> 20
   Gly Xaa Xaa Gly Phe Pro Gly Glu Arg Gly Xaa Xaa
   1 5 10
<210> 21
   <211> 12
   <212> PRT
   <213> Artificial Sequence
<220>
   <223> Synthetic peptide
<220>
   <221> MISC_FEATURE
   <222> (2)..(3)
   <223> Xaa is any amino acid
<220>
   <221> MISC_FEATURE
   <222> (11)..(12)
   <223> Xaa is any amino acid
<400> 21
   Gly Xaa Xaa Gly Phe Pro Gly Glu Asn Gly Xaa Xaa
   1               5                   10
<210> 22
   <211> 12
   <212> PRT
   <213> Artificial Sequence
<220>
   <223> Synthetic peptide
<220>
   <221> MISC_FEATURE
   <222> (2)..(3)
   <223> Xaa is any amino acid
<220>
   <221> MISC_FEATURE
   <222> (11)..(12)
   <223> Xaa is any amino acid
<400> 22
   Gly Xaa Xaa Gly Phe Pro Gly Glu Lys Gly Xaa Xaa
   1               5                   10
<210> 23
   <211> 6
   <212> PRT
   <213> Artificial Sequence
<220>
   <223> synthetic peptide
<220>
   <221> MISC_FEATURE
   <222> (2)..(2)
   <223> Xaa is Lau, Arg or Phe
<220>
   <221> MISC_FEATURE
   <222> (3)..(3)
   <223> Xaa is Pro
<220>
   <221> MISC_FEATURE
   <222> (5)..(5)
   <223> Xaa is Glu
<220>
   <221> MISC_FEATURE
   <222> (6)..(6)
   <223> xaa is Arg, Lys or Asn
<400> 23
   Gly Xaa Xaa Gly Xaa Xaa
   1               5
<210> 24
   <211> 12
   <212> PRT
   <213> Artificial Sequence
<220>
   <223> Synthetic peptide
<220>
   <221> MISC_FEATURE
   <222> (2)..(3)
   <223> Xaa is any amino acid
<220>
   <221> MISC_FEATURE
   <222> (11)..(12)
   <223> xaa is any amino acid
<400> 24
   Gly Xaa Xaa Gly Leu Pro Gly Glu Arg Gly Xaa Xaa
   1               5                   10
<210> 25
   <211> 18
   <212> PRT
   <213> Artificial Sequence
<220>
   <223> Synthetic peptide
<220>
   <221> MISC_FEATURE
   <222> (2)..(3)
   <223> Xaa is any amino acid
<220>
   <221> MISC_FEATURE
   <222> (11)..(12)
   <223> Xaa is any amino acid
<400> 25
   Gly Xaa Xaa Gly Leu Pro Gly Glu Arg Gly Xaa Xaa Gly Leu Pro Gly
   1               5                   10                  15
   Glu Arg
<210> 26
   <211> 27
   <212> PRT
   <213> Artificial Sequence
<220>
   <223> Synthetic peptide
<220>
   <221> MISC_FEATURE
   <222> (2)..(3)
   <223> Xaa is any amino acid
<220>
   <221> MISC-FEATURE
   <222> (11)..(12)
   <223> xaa is any amino acid
<220>
   <221> misc_feature
   <222> (19)..(19)
   <223> Xaa can be any naturally occurring amino acid
<220>
   <221> MISC_FEATURE
   <222> (20)..(21)
   <223> Xaa is any amino acid
<400> 26
   Gly Xaa Xaa Gly Leu Pro Gly Glu Arg Gly Xaa Xaa Gly Leu Pro Gly
   1               5                   10                  15
   Glu Arg Gly Xaa Xaa Gly Leu Pro Gly Glu Arg
               20                  25